(19) Europäisches Patentamt
European Patent Office
Office européen des brevets

(11) Publication number : **0 463 873 A1**

(12) **EUROPEAN PATENT APPLICATION**

(21) Application number : **91305804.6**

(22) Date of filing : **27.06.91**

(51) Int. Cl.$^5$ : **C07D 417/04,** C07D 417/14, A61K 31/44, A61K 31/445

(30) Priority : **27.06.90 JP 169479/90**

(43) Date of publication of application : **02.01.92 Bulletin 92/01**

(84) Designated Contracting States : **AT BE CH DE DK ES FR GB GR IT LI LU NL SE**

(71) Applicant : **Sankyo Company Limited**
**5-1 Nihonbashi Honcho 3-chome Chuo-ku**
**Tokyo (JP)**

(72) Inventor : **Fukumi, Hiroshi**
**c/o Sankyo Company Ltd., 2-58, Hiromachi 1-chome**
**Shinagawa-ku, Tokyo 140 (JP)**
Inventor : **Sakamoto, Toshiaki**
**c/o Sankyo Company Ltd., 2-58, Hiromachi 1-chome**
**Shinagawa-ku, Tokyo 140 (JP)**
Inventor : **Sugiyama, Mitsuo**
**c/o Sankyo Company Ltd., 2-58, Hiromachi 1-chome**
**Shinagawa-ku, Tokyo 140 (JP)**
Inventor : **Oshima, Takeshi**
**c/o Sankyo Company Ltd., 2-58, Hiromachi 1-chome**
**Shinagawa-ku, Tokyo 140 (JP)**
Inventor : **Iijima, Yasuteru**
**c/o Sankyo Company Ltd., 2-58, Hiromachi 1-chome**
**Shinagawa-ku, Tokyo 140 (JP)**
Inventor : **Yamaguchi, Takeshi**
**c/o Sankyo Company Ltd., 2-58, Hiromachi 1-chome**
**Shinagawa-ku, Tokyo 140 (JP)**

(74) Representative : **Gibson, Christian John Robert et al**
**MARKS & CLERK 57/60 Lincoln's Inn Fields**
**London WC2A 3LS (GB)**

(54) **Thiazolidinecarboxylic acid amide derivatives having anti-allergic activity, their preparation and their use.**

(57) Compounds of formula (I) :

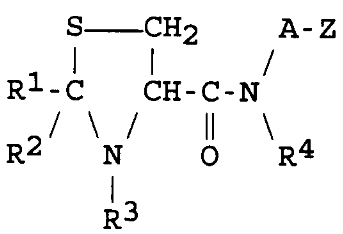

(I)

[in which $R^1$ is optionally substituted pyridyl ; $R^2$ is hydrogen, alkyl or optionally substituted pyridyl ; $R^3$ is hydrogen or various organic groups ; $R^4$ is hydrogen or alkyl ; A is alkylene ; and Z is a substituted piperidyl, piperazinyl or homopiperazinyl group which is substituted at least by a di-substituted methyl, methoxy or methylene group, each substituent being phenyl or heterocyclyl] ; and pharmaceutically acceptable salts thereof have anti-allergic, anti-asthma and anti-PAF activities. Methods of making the compounds are also provided.

EP 0 463 873 A1

The present invention relates to a series of new thiazolidinecarboxylic acid amide derivatives which not only have anti-allergic and anti-asthmatic activities but also have anti-PAF activity. The invention also provides methods and compositions using these compounds as well as processes for their preparation.

A number of compounds having anti-allergic activities are known, and it is also known that compounds having a heterocyclylalkylamide structure have anti-allergic activity [see, for example, US Patent No. 4 965 266, Chemical Pharmaceutical Bulletin, 37, p. 1256 (1989), etc.].

PAF (platelet activating factor) exhibits a strong platelet activating and aggregating effect, from which it derives its name. It has, however, in recent years been seen to be a potentially crucial mediator in a wide variety of pathological processes. Thus, it also has a hypotensive effect and increases vasopermeability; it is believed to be an active agent in the induction of the shock state (for example endotoxin-induced shock or anaphylactic shock) and to act as a mediator of inflammatory disease. It has also been found to play an important role in nephritis, myocardial infarction, angina pectoris, asthma, cardiac and systemic anaphylaxis, gastric and intestinal ulceration, psoriasis and immune and renal disorders.

However, although it has recently appeared to us that it would be desirable to develop anti-allergic agents which not only have anti-allergic activity but also have activities which cooperate with the anti-allergic activity, such as PAF antagonism, no drugs satisfying this demand have yet been put on the market.

For example, while it is known that certain thiazolidine derivatives, for example Compound (A) shown below (which is described in US Patent No. 4 987 132), have PAF antagonism, their anti-allergic activity is very weak. Other prior art compounds described in the same US Patent have anti-PAF activities, but their anti-allergic activity is either also very weak or effectively non-existent.

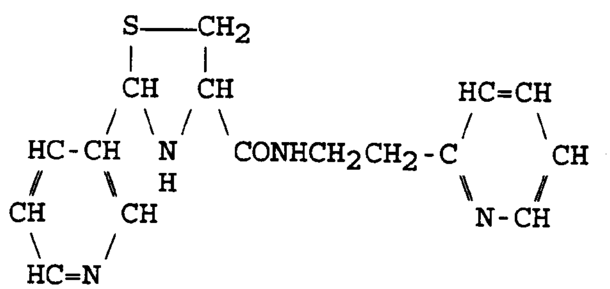

(A)

We have now surprisingly found a series of compounds which combine strong anti-allergic, anti-asthmatic and anti-PAF activities, all in the same compound, a combination of activities that, so far as we are aware, has not hitherto been known. Moreover, the compounds of the invention have sufficiently low toxicities to render them of potential value for the treatment and prophylaxis of disorders in, inter alia, human beings.

The compounds of the present invention are those thiazolidinecarboxylic acid amide derivatives of formula (I):

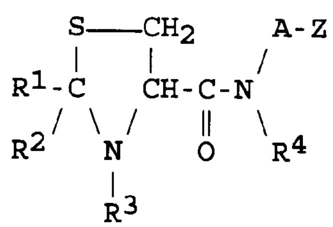

(I)

in which:

$R^1$ represents a pyridyl group which is unsubstituted or which is substituted by at least one alkyl substituent having from 1 to 4 carbon atoms and/or alkoxy substituent having from 1 to 4 carbon atoms;

$R^2$ represents a hydrogen atom, an alkyl group having from 1 to 4 carbon atoms, or a pyridyl group which is unsubstituted or which is substituted by at least one alkyl substituent having from 1 to 4 carbon atoms and/or alkoxy substituent having from 1 to 4 carbon atoms;

$R^3$ represents a hydrogen atom, an alkyl group having from 1 to 4 carbon atoms, an alkoxycarbonyl group having from 2 to 5 carbon atoms, an aralkyloxycarbonyl group in which the alkyl part has from 1 to 4 carbon atoms and the aryl part is as defined below, an aryloxycarbonyl group in which the aryl part is as defined below, an aliphatic carboxylic acyl group having from 1 to 5 carbon atoms, an aliphatic carboxylic acyl group which has

from 2 to 5 carbon atoms and which is substituted by at least one halogen atom, an arylcarbonyl group in which the aryl part is as defined below, an alkylsulphonyl group having from 1 to 4 carbon atoms, an arylsulphonyl group in which the aryl part is as defined below, or a group of formula $-B-NR^5R^6$, in which

B represents an alkylene or alkylidene group having from 2 to 4 carbon atoms; and

$R^5$ and $R^6$ are the same or different and each represents a hydrogen atom or an alkyl group having from 1 to 4 carbon atoms;

said aryl groups have from 6 to 10 ring atoms and are unsubstituted or are substituted by at least one substituent selected from alkyl groups having from 1 to 4 carbon atoms, alkoxy groups having from 1 to 4 carbon atoms and halogen atoms;

$R^4$ represents a hydrogen atom or an alkyl group having from 1 to 4 carbon atoms;

A represents an alkylene or alkylidene group having from 2 to 7 carbon atoms; and

Z represents a group of formula:

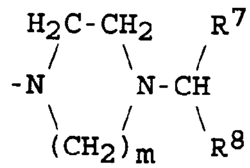

(II)

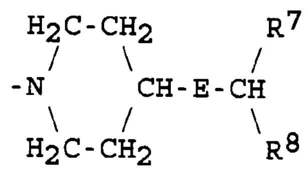

(III)

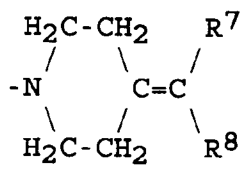

(IV)

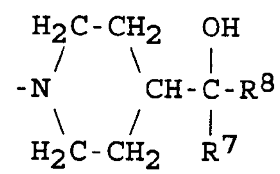

(V)

or a group of formula (II), (III), (IV) or (V) in which one or more of the ring atoms is substituted by an alkyl group having from 1 to 4 carbon atoms;

in which:

$R^7$ and $R^8$ are the same or different and each represents an unsubstituted phenyl group, a substituted phenyl group which is substituted by at least one of substituents (a), defined below, or an aromatic heterocyclic group which has 5 or 6 ring atoms of which 1 or 2 are nitrogen and/or oxygen and/or sulphur hetero-atoms, said aromatic heterocyclic group being unsubstituted or being substituted by at least one of substituents (a), defined below;

said substituents (a) are selected from alkyl groups having from 1 to 4 carbon atoms, alkoxy groups having from 1 to 4 carbon atoms, halogen atoms and haloalkyl groups having from 1 to 4 carbon atoms;

E represents a direct carbon-carbon single bond or an oxygen atom (-O-); and

m is 2 or 3;

and pharmaceutically acceptable salts thereof.

The invention also provides a composition for the treatment or prophylaxis of histamine- or PAF- related disorders, such as allergies or asthma, in a mammal, e.g. a human being, which comprises an effective amount of an anti-histamine or anti-PAF agent in admixture with a pharmaceutically acceptable carrier or diluent, wherein the anti-histamine or anti-PAF agent is at least one compound of formula (I) or a pharmaceutically acceptable salt thereof.

The invention also provides the use of compounds of formula (I) and pharmaceutically acceptable salts thereof in therapy.

The invention still further provides the use of a compound of formula (I) or a pharmaceutically acceptable salt thereof for the manufacture of a medicament for the treatment or prophylaxis of histamine-related disorders, such as allergies or asthma, in a mammal, e.g. a human being.

The invention also provides novel processes for the preparation of the compounds of the present invention, which processes are described in more detail hereafter.

In the compounds of the present invention, where the substituent on the pyridyl group represented by $R^1$ or $R^2$ is an alkyl group or where $R^2$, $R^3$, $R^4$, $R^5$, $R^6$, substituent (a) or the substituent on aryl groups or said groups of formulae (II), (III), (IV) or (V) is an alkyl group, this may be a straight or branched chain alkyl group having from 1 to 4 carbon atoms. Examples of such groups include the methyl, ethyl, propyl, isopropyl, butyl, isobutyl, sec-butyl and t-butyl groups, of which the methyl, ethyl, propyl, isopropyl, butyl and sec-butyl groups are preferred, the methyl and ethyl groups being more preferred, and the methyl group being most preferred.

Where the substituent on the pyridyl group represented by $R^1$ or $R^2$ is an alkoxy group or where substituent (a) or the substituent on aryl groups is an alkoxy group, this may be a straight or branched chain alkoxy group having from 1 to 4 carbon atoms. Examples of such groups include the methoxy, ethoxy, propoxy, isopropoxy, butoxy, isobutoxy, sec-butoxy and t-butoxy groups, of which the methoxy, ethoxy, propoxy, isopropoxy, butoxy and isobutoxy groups are preferred, the methoxy and ethoxy groups being more preferred, and the methoxy group being most preferred.

$R^1$ is preferably an unsubstituted pyridyl group or a substituted pyridyl group having at least one substituent selected from alkyl groups which have from 1 to 4 carbon atoms, and more preferably having 0 or 1 such substituent, and is most preferably an unsubstituted pyridyl group.

$R^2$ is preferably a hydrogen atom.

Where $R^3$ represents an alkoxycarbonyl group, the alkoxy part of this may,be a straight or branched chain alkoxy group having from 1 to 4 carbon atoms, i.e. the alkoxycarbonyl group itself has from 2 to 5 carbon atoms. Examples of such groups include the methoxycarbonyl, ethoxycarbonyl, propoxycarbonyl, isopropoxycarbonyl, butoxycarbonyl, isobutoxycarbonyl, sec-butoxycarbonyl and t-butoxycarbonyl groups, of which the methoxycarbonyl, ethoxycarbonyl, propoxycarbonyl, isopropoxycarbonyl, butoxycarbonyl, isobutoxycarbonyl and t-butoxycarbonyl groups are preferred, the methoxycarbonyl and t-butoxycarbonyl groups being most preferred.

Where $R^3$ represents an aralkyloxycarbonyl group, the alkyl part has from 1 to 4 carbon atoms and may be any of the alkyl groups exemplified above. The aryl part is as defined above, and is preferably a phenyl or naphthyl (1- or 2- naphthyl) group, especially a phenyl group, which may be substituted or unsubstituted. If substituted, the group has one or more substituents selected from alkyl and alkoxy groups each having from 1 to 4 carbon atoms (e.g. as exemplified above) and halogen atoms (e.g. fluorine, chlorine, bromine or iodine atoms, preferably fluorine or chlorine atoms). Where the group is substituted, there is, in principle, no restriction on the number of substituents, except such as may be imposed by the number of substitutable positions or possibly by steric constraints. Hence, for a phenyl group, the maximum number of substituents is 5, whilst the maximum number of substituents on a naphthyl group is 7. However, in general, we prefer from 1 to 3 substituents. Similar considerations apply herein wherever substituted groups are referred to and the number of substituents is not otherwise specified. Examples of such aralkyloxycarbonyl groups include the benzyloxycarbonyl, phenethyloxycarbonyl, 1-, 2- and 3-phenylpropoxycarbonyl, 4-phenylbutoxycarbonyl and 1- and 2-naphthylmethoxycarbonyl groups and substituted analogues thereof, such as the 4-chlorobenzyloxycarbonyl, 4-fluorobenzyloxycarbonyl, 3-chlorobenzyloxycarbonyl, 3-fluorobenzyloxycarbonyl, 4-methylbenzyloxycarbonyl, 4-methoxybenzyloxycarbonyl, 3-methylbenzyloxycarbonyl, 3-methoxybenzyloxycarbonyl, 4-ethylbenzyloxycarbonyl, 4-ethoxybenzyloxycarbonyl, 3-ethylbenzyloxycarbonyl, 3-ethoxybenzyloxycarbonyl, 4-propylbenzyloxycarbonyl, 4-propoxybenzyloxycarbonyl, 3-propylbenzyloxycarbonyl, 3-propoxybenzyloxycarbonyl, 4-butylbenzyloxycarbonyl, 4-butoxybenzyloxycarbonyl, 3-butylbenzyloxycarbonyl and 3-butoxybenzyloxycarbonyl groups.

Where $R^3$ represents an aryloxycarbonyl group, the aryl part is as defined above and examples of such groups include the phenoxycarbonyl, 1- and 2- naphthyloxycarbonyl, o-, m- and p-tolyloxycarbonyl, o-, m- and p-methoxyphenoxycarbonyl, o-, m- and p-chlorophenoxycarbonyl and o-, m- and p-fluorophenoxycarbonyl groups, of which the phenoxycarbonyl group is preferred.

Where $R^3$ represents an aliphatic carboxylic acyl group, this may be a straight or branched chain group having from 1 to 5 carbon atoms, and, in the case of those groups which have from 2 to 5 carbon atoms, it may be unsubstituted or substituted by at least one halogen atom, and preferably 0 or from 1 to 3 halogen atoms. The group is preferably an alkanoyl group having from 1 to 5 carbon atoms or a haloalkanoyl group having from

2 to 5 carbon atoms, but it may also be an alkenoyl or alkynoyl group having from 3 to 5 carbon atoms. Examples of the unsubstituted groups include the formyl, acetyl, propionyl, butyryl, isobutyryl, valeryl, isovaleryl, pivaloyl, acryloyl, methacryloyl, propioloyl, crotonoyl and isocrotonoyl groups, of which the formyl, acetyl, propionyl, butyryl, valeryl, isovaleryl and pivaloyl groups are preferred. Examples of the substituted groups include the trifluoroacetyl, chloroacetyl, fluoroacetyl, 3,3,3-trichloropropionyl, 4-chlorobutyryl and 5-fluorovaleryl groups, of which the trifluoroacetyl and chloroacetyl groups are preferred. The acetyl group is most preferred.

Where $R^3$ represents an arylcarbonyl group, the aryl part is as defined above, and examples of such groups include the benzoyl, 1- and 2- naphthoyl, o-, m- and p-toluoyl, o-, m- and p-anisoyl and veratroyl groups, of which the benzoyl group is preferred.

Where $R^3$ represents an alkylsulphonyl group, this may be a straight or branched chain alkylsulphonyl group having from 1 to 4 carbon atoms. Examples of such groups include the methanesulphonyl, ethanesulphonyl, propanesulphonyl, isopropanesulphonyl, butanesulphonyl, isobutanesulphonyl, sec-butanesulphonyl and t-butanesulphonyl groups, of which the methanesulphonyl, ethanesulphonyl, propanesulphonyl, isopropanesulphonyl, butanesulphonyl and sec-butanesulphonyl groups are preferred, the methanesulphonyl and ethanesulphonyl groups being most preferred.

Where $R^3$ represents an arylsulphonyl group, the aryl part is as defined above, and examples of such groups include the benzenesulphonyl, 1- and 2- naphthalenesulphonyl, and o-, m- and p-toluenesulphonyl groups, of which the benzenesulphonyl and p-toluenesulphonyl groups are preferred.

B represents an alkylene or alkylidene group having from 2 to 4 carbon atoms, which may be a straight or branched chain group. Examples of such groups include the ethylene, ethylidene, trimethylene, propylene, isopropylidene, tetramethylene and 2-methyltrimethylene groups, preferably the ethylene group.

$R^3$ is preferably a hydrogen atom, an alkyl group having from 1 to 4 carbon atoms, an alkoxycarbonyl group having from 2 to 5 carbon atoms or an unsubstituted alkanoyl group, most preferably a hydrogen atom.

$R^4$ is most preferably a hydrogen atom.

A represents an alkylene or alkylidene group having from 2 to 7, preferably from 2 to 6, carbon atoms, which may be a straight or branched chain group. Examples of such groups include the ethylene, ethylidene, trimethylene, propylene, isopropylidene, tetramethylene, 2-methyltrimethylene, pentamethylene, hexamethylene and heptamethylene groups, preferably the ethylene, trimethylene and tetramethylene groups, and more preferably the ethylene and trimethylene groups.

Examples of the groups and atoms which may be included in substituents (a) are:

alkyl groups having from 1 to 4 carbon atoms, such as the methyl, ethyl, propyl, isopropyl, butyl, isobutyl, sec-butyl and t-butyl groups, the methyl and ethyl groups being more preferred, and the methyl group being most preferred;

alkoxy groups having from 1 to 4 carbon atoms, such as the methoxy, ethoxy, propoxy, isopropoxy, butoxy, isobutoxy, sec-butoxy and t-butoxy groups, the methoxy and ethoxy groups being more preferred, and the methoxy group being most preferred;

halogen atoms, such as the fluorine, chlorine, bromine and iodine atoms, the fluorine and chlorine atoms being most preferred; and

haloalkyl groups having from 1 to 4 carbon atoms, such as the chloromethyl, fluoromethyl, bromomethyl, iodomethyl, 2-chloroethyl, 2-fluoroethyl, 2-bromoethyl, 2-iodoethyl, 3-chloropropyl, 3-fluoropropyl, 3-bromopropyl, 3-iodopropyl, 4-chlorobutyl, 4-fluorobutyl, 4-bromobutyl, 4-iodobutyl, dichloromethyl, difluoromethyl, dibromomethyl, diiodomethyl, trichloromethyl, trifluoromethyl, tribromomethyl, triiodomethyl, 2,2,2-trichloroethyl and 2,2,2-trifluoroethyl groups, of which the trifluoromethyl group is preferred.

$R^7$ and $R^8$ may represent unsubstituted phenyl groups or substituted phenyl groups which are substituted by at least one of substituents (a). In the case of the substituted groups, there may be one or more substituents, preferably from 1 to 3 substituents, and more preferably 1 substituent. Where there is one substituent, it is preferably at the 4-position of the phenyl group, and, where there is more than one substituent, one of these is preferably at the 4-position of the phenyl group. Preferred examples of such substituted phenyl groups include the chlorophenyl, fluorophenyl, bromophenyl, iodophenyl, 2,4-dichlorophenyl, 2,4-difluorophenyl, tolyl, ethylphenyl, propylphenyl, butylphenyl, methoxyphenyl, ethoxyphenyl, propoxyphenyl, butoxyphenyl and trifluoromethylphenyl groups, the chlorophenyl, fluorophenyl, tolyl, methoxyphenyl and trifluoromethylphenyl groups being more preferred, and the chlorophenyl, fluorophenyl and tolyl groups being most preferred, and, in the case of the mono-substituted groups, these may be o, m- or p-, preferably p-, substituted.

Where $R^7$ or $R^8$ represents an aromatic heterocyclic group, this has 5 or 6 ring atoms. Of these atoms, 1 or 2 are nitrogen and/or oxygen and/or sulphur hetero-atoms. Where there are two hetero-atoms, these may be the same or different and they are selected from nitrogen, oxygen and sulphur atoms; however, more preferably one is a nitrogen atom and the other is a nitrogen, oxygen or sulphur atom. Most preferably, there is a single hetero-atom, and still more preferably this is a nitrogen or sulphur atom. Such groups may be unsubstituted or they may be substituted by at least one (preferably from 1 to 3) of substituents (a), defined and exemplified above, preferably by an alkyl group having from 1 to 4 carbon atoms. Examples of such unsubstituted groups include the furyl, thienyl, pyrrolyl, pyridyl, thiazolyl, isothiazolyl, oxazolyl, isoxazolyl, imidazolyl, pyrazolyl, pyranyl, pyrazinyl, pyridazinyl, pyrimidinyl, H-pyrrolyl and furazanyl groups, preferably the furyl, thienyl and pyridyl groups, and more preferably the thienyl and pyridyl groups. Such groups may be unsubstituted or they may be substituted by at least one of substituents (a), defined and exemplified above.

More preferably either both of $R^7$ and $R^8$ represents an optionally substituted phenyl group or one of them represents an optionally substituted phenyl group and the other represents one of these heterocyclic groups, more preferably a thienyl or pyridyl group.

Examples of groups which may be represented by Z include the 4-(diphenylmethyl)-1-piperazinyl, 4-[α-(fluorophenyl)benzyl]-1-piperazinyl, 4-[α-(chlorophenyl)benzyl]-1-piperazinyl, 4-[bis(fluorophenyl)methyl]-1-piperazinyl, 4-[α-(chlorophenyl)-o-, m- or p-fluorobenzyl]-1-piperazinyl, 4-[α-(fluorophenyl)-o-, m- or p-methylbenzyl]-1-piperazinyl, 4-[α-(fluorophenyl)-o-, m- or p-methoxybenzyl]-1-piperazinyl, 4-[bis(chlorophenyl)methyl]-1-piperazinyl, 4-[α-(chlorophenyl)-o-, m- or p-methylbenzyl]-1-piperazinyl, 4-[α-(chlorophenyl)-o-, m- or p-methoxybenzyl]-1-piperazinyl, 4-[α-(methoxyphenyl)benzyl]-1-piperazinyl, 4-[bis(methoxyphenyl)methyl]-1-piperazinyl, 4-[α-(methylphenyl)benzyl]-1-piperazinyl, 4-[bis(methylphenyl)methyl]-1-piperazinyl, 4-(diphenylmethyl)-2,5-dimethyl-1-piperazinyl, 4-[α-(fluorophenyl)benzyl]-2,5-dimethyl-1-piperazinyl, 4-[bis(fluorophenyl)methyl]-2,5-dimethyl-1-piperazinyl, 4-(diphenylmethyl)-1-homopiperazinyl, 4-[α-(fluorophenyl)benzyl]-1-homopiperazinyl, 4-[bis(fluorophenyl)methyl]-1-homopiperazinyl, 4-[α-(chlorophenyl)benzyl]-1-homopiperazinyl, 4-[bis(chlorophenyl)methyl]-1-homopiperazinyl, 4-[α-(chlorophenyl)-o-, m- or p-fluorobenzyl]-1-homopiperazinyl, 4-[α-(methylphenyl)benzyl]-1-homopiperazinyl, 4-[bis(methylphenyl)methyl]-1-homopiperazinyl, 4-[α-(methoxyphenyl)benzyl]-1-homopiperazinyl, 4-[α-(methoxyphenyl)-o-, m- or p-methylbenzyl]-1-homopiperazinyl, 4-[α-(methoxyphenyl)-o-, m- or p-methylbenzyl]-1-piperazinyl, 4-(diphenylmethyl)-1-piperidyl, 4-[α-(fluorophenyl)benzyl]-1-piperidyl, 4-[bis(fluorophenyl)methyl]-1-piperidyl, 4-[α-(chlorophenyl)benzyl]-1-piperidyl, 4-[bis(chlorophenyl)methyl]-1-piperidyl, 4-[α-(methylphenyl)benzyl]-1-piperidyl, 4-[bis(methylphenyl)methyl]-1-piperidyl, 4-[α-(methoxyphenyl)-o-, m- or p-methylbenzyl]-1-piperidyl, 4-[α-(methoxyphenyl)benzyl]-1-piperidyl, 4-[bis(methoxyphenyl)methyl]-1-piperidyl, 4-(diphenylmethoxy)-1-piperidyl, 4-[α-(fluorophenyl)benzyloxy]-1-piperidyl, 4-[bis(fluorophenyl)methoxy]-1-piperidyl, 4-[α-(chlorophenyl)benzyloxy]-1-piperidyl, 4-[bis(chlorophenyl)methoxy]-1-piperidyl, 4-[α-(chlorophenyl)-o-, m- or p-fluorobenzyloxy]-1-piperidyl, 4-[α-(methylphenyl)benzyloxy]-1-piperidyl, 4-[bis(methylphenyl)methoxy]-1-piperidyl, 4-[α-(methoxyphenyl)-o-, m- or p-methylbenzyloxy]-1-piperidyl, 4-[α-(methoxyphenyl)benzyloxy]-1-piperidyl, 4-(diphenylmethylene)-1-piperidyl, 4-[α-(fluorophenyl)benzylidene]-1-piperidyl, 4-[bis(fluorophenyl)methylene]-1-piperidyl, 4-[α-(chlorophenyl)benzylidene]-1-piperidyl, 4-[bis(chlorophenyl)methylene]-1-piperidyl, 4-[α-(methylphenyl)benzylidene]-1-piperidyl, 4-[bis(methylphenyl)methylene]-1-piperidyl, 4-[α-(methoxyphenyl)-o-, m- or p-methylbenzylidene]-1-piperidyl, 4-[α-(methoxyphenyl)benzylidene]-1-piperidyl, 4-[bis(methoxyphenyl)methylene]-1-piperidyl, 4-(α-hydroxydiphenylmethyl)-1-piperidyl, 4-[α-(fluorophenyl)-α-hydroxybenzyl]-1-piperidyl, 4-[bis(fluorophenyl)-α-hydroxymethyl]-1-piperidyl, 4-[α-(chlorophenyl)-α-hydroxybenzyl]-1-piperidyl, 4-[bis(chlorophenyl)-α-hydroxymethyl]-1-piperidyl, 4-[α-hydroxy-α-(methylphenyl)benzyl]-1-piperidyl, 4-[α-hydroxybis(methylphenyl)methyl]-1-piperidyl and 4-[α-hydroxy-α-(methoxyphenyl)benzyl]-1-piperidyl groups.

More preferred examples of the groups which may be represented by Z include the 4-(diphenylmethyl)-1-piperazinyl, 4-[α-(chlorophenyl)benzyl]-1-piperazinyl, 4-[bis(fluorophenyl)methyl]-1-piperazinyl, 4-[α-(chlorophenyl)-o-, m- or p-fluorobenzyl]-1-piperazinyl, 4-[bis(chlorophenyl)methyl]-1-piperazinyl, 4-(diphenylmethyl)-1-piperidyl, 4-[bis(fluorophenyl)methyl]-1-piperidyl, 4-[α-(chlorophenyl)benzyl]-1-piperidyl, 4-(diphenylmethoxy)-1-piperidyl, 4-[α-(fluorophenyl)benzyloxy]-1-piperidyl, 4-[bis(fluorophenyl)methoxy]-1-piperidyl, 4-[α-(chlorophenyl)benzyloxy]-1-piperidyl, 4-(diphenylmethylene)-1-piperidyl, 4-[α-(fluorophenyl)benzylidene]-1-piperidyl, 4-[bis(fluorophenyl)methylene]-1-piperidyl, 4-[α-(chlorophenyl)benzylidene]-1-piperidyl, 4-(α-hydroxydiphenylmethyl)-1-piperidyl, 4-[α-(fluorophenyl)-α-hydroxybenzyl]-1-piperidyl, 4-[bis(fluorophenyl)-α-hydroxymethyl]-1-piperidyl and 4-[α-(chlorophenyl)-α-hydroxybenzyl]-1-piperidyl groups.

In all of the mono-substituted phenyl groups above which are included among the preferred and more preferred meanings of Z, the substituent may be at the o-, m- or p- position, but is preferably at the p-position.

The compounds of the present invention can form acid addition salts. There is no particular restriction on the nature of these salts, provided that, where they are intended for therapeutic use, they are pharmaceutically acceptable. Where they are intended for non-therapeutic uses, e.g. as intermediates in the preparation of other, and possibly more active, compounds, even this restriction does not apply. Examples of such acid addition salts include: salts with mineral acids, especially hydrohalic acids (such as hydrofluoric acid, hydrobromic acid, hydroiodic acid or hydrochloric acid), nitric acid, perchloric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic

acid; salts with organic carboxylic acids, such as acetic acid, fumaric acid, tartaric acid, oxalic acid, maleic acid, malic acid, succinic acid or citric acid; and salts with amino acids, such as glutamic acid or aspartic acid.

The compounds of the present invention necessarily contain several asymmetric carbon atoms in their molecules, and can thus form optical isomers. Although these are all represented herein by a single molecular formula, the present invention includes both the individual, isolated isomers and mixtures, including racemates thereof. The preferred configuration at the 4-position of the thiazolidine ring is the R configuration. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

A preferred class of compounds of the present invention are those compounds of formula (I) and salts thereof in which:

$R^1$ represents a pyridyl group which is unsubstituted or which is substituted by at least one alkyl substituent having from 1 to 4 carbon atoms;

$R^2$ represents a hydrogen atom;

$R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms;

$R^4$ represents a hydrogen atom or an alkyl group having from 1 to 4 carbon atoms;

A represents an alkylene or alkylidene group having from 2 to 7 carbon atoms; and

Z represents a group of formula (II), (III), (IV) or (V), in which:

$R^7$ and $R^8$ are the same or different and each represents an unsubstituted phenyl group, a substituted phenyl group which is substituted by at least one of substituents (a′), defined below, or an aromatic heterocyclic group which has 5 or 6 ring atoms of which 1 is a nitrogen and/or oxygen and/or sulphur hetero-atom, said aromatic heterocyclic group being unsubstituted or being substituted by at least one alkyl substituent having from 1 to 4 carbon atoms;

said substituents (a′) are selected from alkyl groups having from 1 to 4 carbon atoms, halogen atoms and trifluoromethyl groups;

E represents an oxygen atom; and

m is 2.

Still more preferred compounds of the present invention are those compounds of formula (I) and salts thereof in which:

$R^1$ represents an unsubstituted pyridyl group;

$R^2$ represents a hydrogen atom;

$R^3$ represents a hydrogen atom;

$R^4$ represents a hydrogen atom or a methyl or ethyl group;

A represents an alkylene or alkylidene group having from 2 to 4 carbon atoms; and

Z represents a group of formula (II), (III), (IV) or (V), in which:

$R^7$ and $R^8$ are the same or different and each represents an unsubstituted phenyl group, a substituted phenyl group which is substituted by at least one of substituents (a″), defined below, a pyridyl group or a thienyl group;

said substituents (a″) are selected from methyl groups, fluorine atoms and chlorine atoms;

E represents an oxygen atom; and

m is 2.

Another preferred class of compounds of the present invention are those compounds of formula (I) and salts thereof in which:

$R^1$ represents a pyridyl group which is unsubstituted or which is substituted by at least one alkyl substituent having from 1 to 4 carbon atoms;

$R^2$ represents a hydrogen atom;

$R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms;

$R^4$ represents a hydrogen atom or an alkyl group having from 1 to 4 carbon atoms;

A represents an alkylene or alkylidene group having from 2 to 6 carbon atoms; and

Z represents a group of formula (II), (III), (IV) or (V), in which:

$R^7$ and $R^8$ are the same or different and each represents an unsubstituted phenyl group or a substituted phenyl group which is substituted by at least one of substituents (a‴), defined below;

said substituents (a‴) are selected from alkyl groups having from 1 to 4 carbon atoms and halogen atoms;

E represents a direct carbon-carbon single bond or an oxygen atom; and

m is 2 or 3.

Another still more preferred class of compounds of the present invention are those compounds of formula

(I) and salts thereof in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a group of formula (II), (III), (IV) or (V), in which:

$R^7$ and $R^8$ are the same or different and each represents an unsubstituted phenyl group or a substituted phenyl group which is substituted by at least one of substituents (a″), defined above;

E represents an oxygen atom; and

m is 2.

Most preferred are those compounds of formula (I) and salts thereof in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a 4-[α-(chlorophenyl)benzyl]-1-piperazinyl, 4-[bis(fluorophenyl)methyl]-1-piperazinyl, 4-(diphenylmethylene)-1-piperidyl, 4-[bis(fluorophenyl)methoxy]-1-piperidyl or 4-(α-hydroxydiphenylmethyl)-1-piperidyl group.

Examples of certain of the compounds of the present invention are shown by the following formulae (I-1) to (I-4), in which the symbols used in the formulae are as defined in the respective one of Tables 1 to 4, that is Table 1 relates to formula (I-1), Table 2 relates to formula (I-2), and so on. In the Tables, the following abbreviations are used:

| | |
|---|---|
| Ac | acetyl |
| tBoc | t-butoxycarbonyl |
| DMA | 2-(dimethylamino)ethyl |
| Et | ethyl |
| Fo | formyl |
| Me | methyl |
| Mec | methoxycarbonyl |
| Ph | phenyl |
| Py | pyridyl |
| Tfm | trifluoromethyl |
| Thi | thienyl |

In Table 2, in the column for E, a dash (-) means a direct carbon-carbon single bond and "O" means an oxygen atom.

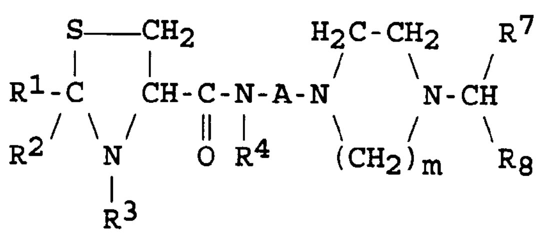

(I-1)

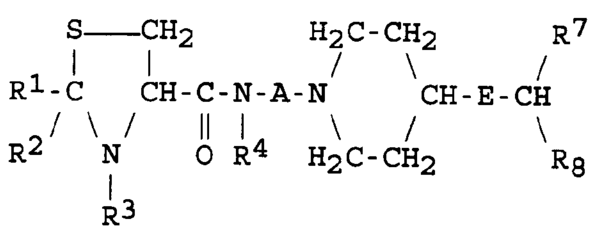

(I-2)

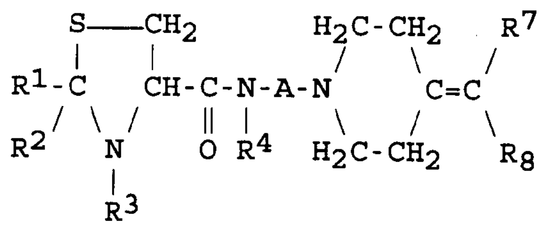

(I-3)

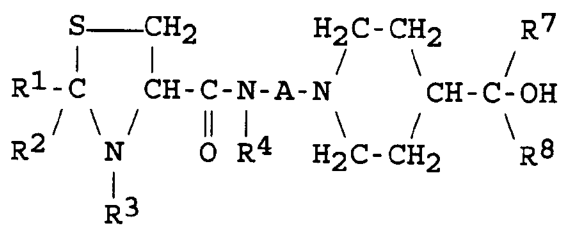

(I-4)

Table 1

| Cpd. | $R^1$ | $R^2$ | $R^3$ | $R^4$ | m | $R^7$ | $R^8$ | A |
|---|---|---|---|---|---|---|---|---|
| 1-1 | 3-Py | H | tBoc | H | 2 | Ph | Ph | $-(CH_2)_2-$ |
| 1-2 | 3-Py | H | tBoc | H | 2 | Ph | Ph | $-(CH_2)_3-$ |
| 1-3 | 3-Py | H | tBoc | H | 2 | Ph | Ph | $-(CH_2)_4-$ |
| 1-4 | 3-Py | H | tBoc | H | 2 | 4-FPh | 4-FPh | $-(CH_2)_2-$ |
| 1-5 | 3-Py | H | tBoc | H | 2 | 4-FPh | 4-FPh | $-(CH_2)_3-$ |
| 1-6 | 3-Py | H | tBoc | H | 2 | 4-FPh | 4-FPh | $-(CH_2)_4-$ |
| 1-7 | 3-Py | H | tBoc | H | 2 | Ph | 4-ClPh | $-(CH_2)_3-$ |
| 1-8 | 3-Py | H | Mec | H | 2 | Ph | Ph | $-(CH_2)_2-$ |
| 1-9 | 3-Py | H | Mec | H | 2 | Ph | Ph | $-(CH_2)_3-$ |
| 1-10 | 3-Py | H | Mec | H | 2 | Ph | Ph | $-(CH_2)_4-$ |
| 1-11 | 3-Py | H | Mec | H | 2 | 4-FPh | 4-FPh | $-(CH_2)_2-$ |
| 1-12 | 3-Py | H | H | H | 2 | 4-FPh | 4-FPh | $-(CH_2)_3-$ |
| 1-13 | 3-Py | H | H | H | 2 | 4-FPh | 4-FPh | $-(CH_2)_4-$ |
| 1-14 | 3-Py | H | H | H | 2 | 4-FPh | 4-FPh | $-(CH_2)_5-$ |
| 1-15 | 3-Py | H | H | H | 2 | 4-FPh | 3-FPh | $-(CH_2)_3-$ |
| 1-16 | 2-Py | H | H | H | 2 | Ph | 4-FPh | $-(CH_2)_3-$ |
| 1-17 | 3-Py | H | H | H | 2 | 4-FPh | 4-ClPh | $-(CH_2)_2-$ |
| 1-18 | 3-Py | H | H | H | 2 | 4-FPh | 4-MePh | $-(CH_2)_3-$ |
| 1-19 | 3-Py | H | H | H | 2 | 4-FPh | 4-MeOPh | $-(CH_2)_4-$ |
| 1-20 | 4-Py | H | H | H | 2 | 4-ClPh | 3-ClPh | $-(CH_2)_3-$ |
| 1-21 | 3-Py | H | H | H | 2 | 3-ClPh | 4-MePh | $-(CH_2)_5-$ |
| 1-22 | 3-Py | H | H | H | 2 | 4-ClPh | 4-MeOPh | $-(CH_2)_3-$ |
| 1-23 | 3-Py | H | H | H | 2 | Ph | 4-MeOPh | $-(CH_2)_2-$ |
| 1-24 | 3-Py | H | H | H | 2 | 4-MeOPh | 4-MeOPh | $-(CH_2)_3-$ |
| 1-25 | 4-Py | H | H | H | 2 | Ph | 4-MePh | $-(CH_2)_2-$ |
| 1-26 | 3-Py | H | H | H | 2 | 4-MePh | 4-MePh | $-(CH_2)_4-$ |
| 1-27 | 5,6-diMe--3-Py | H | H | H | 2 | 4-FPh | 4-FPh | $-(CH_2)_3-$ |
| 1-28 | 3-Py | H | H | H | 2 | Ph | 2-FPh | $-(CH_2)_3-$ |

Table 1 (cont)

| Cpd. | $R^1$ | $R^2$ | $R^3$ | $R^4$ | m | $R^7$ | $R^8$ | A |
|---|---|---|---|---|---|---|---|---|
| 1-29 | 2-Me--3-Py | H | H | H | 2 | Ph | Ph | $-(CH_2)_2-$ |
| 1-30 | 2-Py | H | H | H | 2 | 4-FPh | 4-FPh | $-(CH_2)_3-$ |
| 1-31 | 2-Py | H | H | H | 2 | Ph | Ph | $-(CH_2)_3-$ |
| 1-32 | 3-Py | H | H | H | 2 | Ph | 3-ClPh | $-(CH_2)_3-$ |
| 1-33 | 2-Me--6-Py | H | H | H | 2 | 4-FPh | 4-FPh | $-(CH_2)_3-$ |
| 1-34 | 3-Py | H | Me | H | 2 | 4-FPh | 4-FPh | $-(CH_2)_3-$ |
| 1-35 | 3-Py | H | Fo | H | 2 | 4-FPh | 4-FPh | $-(CH_2)_4-$ |
| 1-36 | 3-Py | H | Ac | H | 2 | Ph | Ph | $-(CH_2)_2-$ |
| 1-37 | 3-Py | H | H | H | 2 | Ph | Ph | $-(CH_2)_3-$ |
| 1-38 | 3-Py | H | DMA | H | 2 | 4-FPh | 4-FPh | $-(CH_2)_3-$ |
| 1-39 | 3-Py | H | H | H | 3 | Ph | Ph | $-(CH_2)_2-$ |
| 1-40 | 4-Py | H | H | H | 3 | Ph | Ph | $-(CH_2)_3-$ |
| 1-41 | 3-Py | H | H | H | 3 | 4-FPh | 4-FPh | $-(CH_2)_3-$ |
| 1-42 | 2-Py | H | H | H | 3 | 4-FPh | 4-FPh | $-(CH_2)_4-$ |
| 1-43 | 3-Py | H | H | H | 3 | Ph | 4-ClPh | $-(CH_2)_5-$ |
| 1-44 | 4-Py | H | H | H | 3 | 4-ClPh | 4-ClPh | $-(CH_2)_2-$ |
| 1-45 | 3-Py | H | H | H | 3 | 4-FPh | 4-ClPh | $-(CH_2)_3-$ |
| 1-46 | 3-Py | H | H | H | 3 | Ph | 4-MePh | $-(CH_2)_3-$ |
| 1-47 | 3-Py | H | H | H | 3 | 4-MePh | 4-MePh | $-(CH_2)_2-$ |
| 1-48 | 3-Py | H | H | H | 3 | 4-MeOPh | 4-MeOPh | $-(CH_2)_4-$ |
| 1-49 | 3-Py | H | H | H | 2 | 4-FPh | 3-FPh | $-(CH_2)_3-$ |
| 1-50 | 3-Py | H | H | H | 2 | Ph | 2-FPh | $-(CH_2)_3-$ |
| 1-51 | 3-Py | H | H | H | 2 | Ph | 3-ClPh | $-(CH_2)_3-$ |
| 1-52 | 3-Py | H | H | H | 2 | 4-FPh | 3-MePh | $-(CH_2)_3-$ |
| 1-53 | 3-Py | H | H | H | 2 | 4-FPh | 2-MeOPh | $-(CH_2)_3-$ |
| 1-54 | 3-Py | H | H | H | 2 | Ph | Ph | $-CH_2CH(Me)CH_2-$ |

Table 1 (cont)

| Cpd. | $R^1$ | $R^2$ | $R^3$ | $R^4$ | m | $R^7$ | $R^8$ | A |
|---|---|---|---|---|---|---|---|---|
| 1-55 | 3-Py | H | H | H | 2 | Ph | 4-ClPh | $-CH_2CH(Me)CH_2-$ |
| 1-56 | 3-Py | H | H | H | 2 | Ph | 4-FPh | $-CH_2CH(Me)CH_2-$ |
| 1-57 | 3-Py | H | H | H | 2 | 4-FPh | 4-FPh | $-CH_2CH(Me)CH_2-$ |
| 1-58 | 3-Py | H | H | H | 3 | Ph | Ph | $-CH_2CH(Me)CH_2-$ |
| 1-59 | 3-Py | H | tBoc | H | 2 | Ph | Ph | $-CH_2CH(Me)CH_2-$ |
| 1-60 | 3-Py | H | Me | Me | 2 | Ph | Ph | $-(CH_2)_3-$ |
| 1-61 | 3-Py | H | Me | Me | 2 | 4-FPh | 4-FPh | $-(CH_2)_3-$ |
| 1-62 | 3-Py | 3-Py | H | H | 2 | 4-FPh | 4-FPh | $-(CH_2)_3-$ |
| 1-63* | 3-Py | H | H | H | 2 | Ph | Ph | $-(CH_2)_3-$ |
| 1-64 | 3-Py | H | H | H | 2 | Ph | 2-Py | $-(CH_2)_2-$ |
| 1-65 | 3-Py | H | H | H | 2 | Ph | 2-Py | $-(CH_2)_3-$ |
| 1-66 | 3-Py | H | H | H | 2 | Ph | 2-Py | $-(CH_2)_4-$ |
| 1-67 | 3-Py | H | H | H | 2 | Ph | 2-Py | $-(CH_2)_5-$ |
| 1-68 | 3-Py | H | Me | H | 2 | Ph | 2-Py | $-(CH_2)_2-$ |
| 1-69 | 3-Py | H | Me | H | 2 | Ph | 2-Py | $-(CH_2)_3-$ |
| 1-70 | 3-Py | H | H | H | 2 | 4-FPh | 2-Py | $-(CH_2)_2-$ |
| 1-71 | 3-Py | H | H | H | 2 | 4-FPh | 2-Py | $-(CH_2)_3-$ |
| 1-72 | 3-Py | H | H | H | 2 | 4-ClPh | 2-Py | $-(CH_2)_2-$ |
| 1-73 | 3-Py | H | H | H | 2 | 4-ClPh | 2-Py | $-(CH_2)_4-$ |
| 1-74 | 3-Py | H | H | H | 2 | 4-MePh | 2-Py | $-(CH_2)_3-$ |
| 1-75 | 3-Py | H | H | H | 2 | 4-TfmPh | 2-Py | $-(CH_2)_2-$ |
| 1-76 | 3-Py | H | H | H | 3 | Ph | 2-Py | $-(CH_2)_2-$ |
| 1-77 | 3-Py | H | H | H | 3 | Ph | 2-Py | $-(CH_2)_4-$ |
| 1-78 | 4-Py | H | H | H | 2 | Ph | 2-Py | $-(CH_2)_2-$ |
| 1-79 | 3-Py | H | H | H | 2 | Ph | 3-Py | $-(CH_2)_2-$ |
| 1-80 | 3-Py | H | H | H | 2 | 4-ClPh | 3-Py | $-(CH_2)_3-$ |
| 1-81 | 3-Py | H | H | H | 2 | 4-FPh | 3-Py | $-(CH_2)_4-$ |
| 1-82 | 4-Py | H | H | H | 2 | Ph | 3-Py | $-(CH_2)_2-$ |

Table 1 (cont)

| Cpd. | $R^1$ | $R^2$ | $R^3$ | $R^4$ | m | $R^7$ | $R^8$ | A |
|---|---|---|---|---|---|---|---|---|
| 1-83 | 3-Py | H | H | H | 3 | Ph | 3-Py | $-(CH_2)_2-$ |
| 1-84 | 3-Py | H | H | H | 2 | Ph | 4-Py | $-(CH_2)_2-$ |
| 1-85 | 3-Py | H | H | H | 2 | Ph | 4-Py | $-(CH_2)_4-$ |
| 1-86 | 3-Py | H | H | Me | 2 | Ph | Ph | $-(CH_2)_2-$ |
| 1-87 | 3-Py | H | H | H | 2 | Ph | Ph | $-(CH_2)_2-$ |
| 1-88 | 3-Py | H | H | H | 2 | Ph | 4-ClPh | $-(CH_2)_3-$ |
| 1-89 | 3-Py | H | H | H | 2 | Ph | 4-ClPh | $-(CH_2)_2-$ |
| 1-90 | 3-Py | H | H | H | 2 | Ph | Ph | $-(CH_2)_4-$ |
| 1-91 | 3-Py | H | H | H | 2 | 4-FPh | 4-FPh | $-(CH_2)_2-$ |

* In Compound No. 1-63, the piperazinyl group has methyl substituents at the 2- and 5- positions.

Table 2

| Cpd. | $R^1$ | $R^2$ | $R^3$ | $R^4$ | E | $R^7$ | $R^8$ | A |
|---|---|---|---|---|---|---|---|---|
| 2-1 | 3-Py | H | H | H | - | Ph | Ph | $-(CH_2)_2-$ |
| 2-2 | 3-Py | H | H | H | - | Ph | 4-FPh | $-(CH_2)_4-$ |
| 2-3 | 3-Py | H | H | H | - | 4-FPh | 3-FPh | $-(CH_2)_3-$ |
| 2-4 | 3-Py | H | H | H | - | Ph | 4-ClPh | $-(CH_2)_2-$ |
| 2-5 | 3-Py | H | H | H | - | Ph | 4-ClPh | $-(CH_2)_3-$ |
| 2-6 | 3-Py | H | H | H | - | 4-ClPh | 4-ClPh | $-(CH_2)_4-$ |
| 2-7 | 3-Py | H | H | H | - | Ph | 4-MePh | $-(CH_2)_3-$ |
| 2-8 | 3-Py | H | H | H | - | 4-MePh | 4-MePh | $-(CH_2)_2-$ |
| 2-9 | 3-Py | H | H | H | - | 4-MePh | 4-MeOPh | $-(CH_2)_3-$ |
| 2-10 | 3-Py | H | H | H | - | 4-MeOPh | 4-MeOPh | $-(CH_2)_4-$ |
| 2-11 | 2-Py | H | H | H | - | Ph | 4-MeOPh | $-(CH_2)_5-$ |
| 2-12 | 3-Py | H | H | H | O | 4-FPh | 4-FPh | $-(CH_2)_2-$ |
| 2-13 | 3-Py | H | H | H | O | 4-FPh | 4-FPh | $-(CH_2)_3-$ |
| 2-14 | 3-Py | H | H | H | O | Ph | Ph | $-(CH_2)_4-$ |
| 2-15 | 3-Py | H | H | H | O | Ph | 4-FPh | $-(CH_2)_3-$ |
| 2-16 | 3-Py | H | H | H | O | 4-FPh | 4-FPh | $-(CH_2)_2-$ |
| 2-17 | 3-Py | H | H | H | O | 4-FPh | 4-ClPh | $-(CH_2)_3-$ |
| 2-18 | 3-Py | H | H | H | O | 4-ClPh | 4-ClPh | $-(CH_2)_4-$ |
| 2-19 | 3-Py | H | H | H | O | Ph | 4-ClPh | $-(CH_2)_2-$ |
| 2-20 | 2-Py | H | H | H | O | Ph | Ph | $-(CH_2)_3-$ |
| 2-21 | 4-Py | H | H | H | O | Ph | Ph | $-(CH_2)_2-$ |
| 2-22 | 3-Py | H | H | H | O | Ph | 4-MePh | $-(CH_2)_3-$ |
| 2-23 | 3-Py | H | H | H | O | 4-MePh | 4-MePh | $-(CH_2)_2-$ |
| 2-24 | 2-Py | H | H | H | O | Ph | 4-MeOPh | $-(CH_2)_4-$ |
| 2-25 | 2-Py | H | H | H | O | 4-MeOPh | 4-MeOPh | $-(CH_2)_5-$ |
| 2-26 | 3-Py | H | H | H | O | Ph | 2-FPh | $-(CH_2)_3-$ |
| 2-27 | 3-Py | H | H | H | O | 4-FPh | 3-FPh | $-(CH_2)_3-$ |
| 2-28 | 3-Py | H | H | H | O | 2-FPh | 4-ClPh | $-(CH_2)_3-$ |
| 2-29 | 3-Py | H | H | H | O | 4-ClPh | 2-ClPh | $-(CH_2)_3-$ |

Table 2 (cont)

| Cpd. | $R^1$ | $R^2$ | $R^3$ | $R^4$ | E | $R^7$ | $R^8$ | A |
|---|---|---|---|---|---|---|---|---|
| 2-30 | 3-Py | H | H | H | O | Ph | 3-MePh | $-(CH_2)_3-$ |
| 2-31 | 3-Py | H | H | H | O | 4-MePh | 3-MeOPh | $-(CH_2)_3-$ |
| 2-32 | 3-Py | H | H | H | O | Ph | Ph | $-CH_2CH(Me)CH_2-$ |
| 2-33 | 3-Py | H | H | H | O | Ph | 4-ClPh | $-CH_2CH(Me)CH_2-$ |
| 2-34 | 3-Py | H | H | H | O | Ph | 4-FPh | $-CH_2CH(Me)CH_2-$ |
| 2-35 | 3-Py | H | H | H | O | 4-FPh | 4-FPh | $-CH_2CH(Me)CH_2-$ |
| 2-36 | 3-Py | H | H | H | - | Ph | Ph | $-CH_2CH(Me)CH_2-$ |
| 2-37 | 3-Py | 3-Py | H | H | O | 4-FPh | 4-FPh | $-(CH_2)_3-$ |
| 2-38 | 3-Py | H | H | H | - | Ph | Ph | $-(CH_2)_3-$ |
| 2-39 | 4-Py | H | H | H | - | Ph | Ph | $-(CH_2)_3-$ |
| 2-40 | 2-Me--5-Py | H | H | H | - | Ph | Ph | $-(CH_2)_2-$ |
| 2-41 | 2-Me--5-Py | H | H | H | - | Ph | Ph | $-(CH_2)_3-$ |

Table 3

| Cpd. | $R^1$ | $R^2$ | $R^3$ | $R^4$ | $R^7$ | $R^8$ | A |
|---|---|---|---|---|---|---|---|
| 3-1 | 3-Py | H | tBoc | H | Ph | Ph | $-(CH_2)_2-$ |
| 3-2 | 3-Py | H | tBoc | H | Ph | Ph | $-(CH_2)_3-$ |
| 3-3 | 3-Py | H | H | H | Ph | Ph | $-(CH_2)_2-$ |
| 3-4 | 3-Py | H | H | H | Ph | Ph | $-(CH_2)_3-$ |
| 3-5 | 3-Py | H | H | H | Ph | Ph | $-(CH_2)_4-$ |
| 3-6 | 3-Py | H | H | H | Ph | Ph | $-(CH_2)_5-$ |
| 3-7 | 3-Py | H | H | H | Ph | 4-FPh | $-(CH_2)_3-$ |
| 3-8 | 3-Py | H | H | H | 4-FPh | 4-FPh | $-(CH_2)_4-$ |
| 3-9 | 3-Py | H | H | H | Ph | 4-ClPh | $-(CH_2)_3-$ |
| 3-10 | 3-Py | H | H | H | 4-ClPh | 4-ClPh | $-(CH_2)_3-$ |
| 3-11 | 4-Py | H | H | H | Ph | 4-MePh | $-(CH_2)_2-$ |
| 3-12 | 2-Py | H | H | H | 4-MePh | 4-MePh | $-(CH_2)_5-$ |
| 3-13 | 3-Py | H | H | H | 4-MeOPh | 4-MeOPh | $-(CH_2)_3-$ |
| 3-14 | 4-Py | H | H | H | 4-MeOPh | 4-MeOPh | $-(CH_2)_4-$ |
| 3-15 | 2-Py | H | H | H | Ph | 4-MeOPh | $-(CH_2)_2-$ |
| 3-16 | 3-Py | H | Me | H | Ph | Ph | $-(CH_2)_2-$ |
| 3-17 | 3-Py | H | DMA | H | Ph | Ph | $-(CH_2)_3-$ |
| 3-18 | 3-Py | H | Me | H | Ph | Ph | $-(CH_2)_3-$ |
| 3-19 | 2-Me-<br>-6-Py | H | H | H | Ph | Ph | $-(CH_2)_3-$ |
| 3-20 | 2-Py | H | H | H | Ph | Ph | $-(CH_2)_3-$ |
| 3-21 | 3-Py | H | H | H | Ph | Ph | $-(CH_2)_6-$ |
| 3-22 | 3-Py | H | H | H | 4-FPh | 3-FPh | $-(CH_2)_3-$ |
| 3-23 | 3-Py | H | H | H | Ph | 3-FPh | $-(CH_2)_3-$ |
| 3-24 | 3-Py | H | H | H | Ph | 3-ClPh | $-(CH_2)_3-$ |
| 3-25 | 3-Py | H | H | H | 4-ClPh | 2-ClPh | $-(CH_2)_3-$ |
| 3-26 | 3-Py | H | H | H | Ph | 3-MePh | $-(CH_2)_3-$ |
| 3-27 | 3-Py | H | H | H | 4-MePh | 3-MeOPh | $-(CH_2)_3-$ |
| 3-28 | 3-Py | H | H | H | Ph | Ph | $-CH_2CH(Me)CH_2-$ |

Table 3 (cont)

| Cpd. | $R^1$ | $R^2$ | $R^3$ | $R^4$ | $R^7$ | $R^8$ | A |
|---|---|---|---|---|---|---|---|
| 3-29 | 3-Py | H | H | H | Ph | 4-FPh | $-CH_2CH(Me)CH_2-$ |
| 3-30 | 3-Py | H | H | H | 4-FPh | 4-FPh | $-CH_2CH(Me)CH_2-$ |
| 3-31 | 3-Py | 3-Py | H | H | Ph | Ph | $-(CH_2)_3-$ |
| 3-32 | 3-Py | H | Me | Me | Ph | Ph | $-(CH_2)_3-$ |
| 3-33 | 3-Py | H | Me | Me | 4-FPh | 4-FPh | $-(CH_2)_3-$ |
| 3-34 | 3-Py | H | H | H | 4-FPh | 4-FPh | $-(CH_2)_2-$ |
| 3-35 | 3-Py | H | H | H | 4-FPh | 4-FPh | $-(CH_2)_3-$ |
| 3-36 | 3-Py | H | H | H | Ph | Ph | $-(CH_2)_7-$ |
| 3-37 | 3-Py | H | H | H | 4-FPh | 4-FPh | $-(CH_2)_7-$ |
| 3-38 | 3-Py | H | Me | H | 4-FPh | 4-FPh | $-(CH_2)_2-$ |
| 3-39 | 3-Py | H | Me | H | 4-FPh | 4-FPh | $-(CH_2)_3-$ |
| 3-40 | 3-Py | H | H | H | Ph | 3-Thi | $-(CH_2)_2-$ |
| 3-41 | 3-Py | H | H | H | Ph | 2-Thi | $-(CH_2)_3-$ |
| 3-42 | 2-Me-<br>-5-Py | H | H | H | 4-FPh | 4-FPh | $-(CH_2)_2-$ |
| 3-43 | 2-Me-<br>-5-Py | H | H | H | 4-FPh | 4-FPh | $-(CH_2)_3-$ |
| 3-44 | 2-Me-<br>-5-Py | H | H | H | Ph | Ph | $-(CH_2)_2-$ |
| 3-45 | 2-Me-<br>-5-Py | H | H | H | Ph | Ph | $-(CH_2)_3-$ |
| 3-46 | 4-Py | H | H | H | Ph | Ph | $-(CH_2)_3-$ |
| 3-47 | 2-Py | H | H | Me | Ph | Ph | $-(CH_2)_2-$ |
| 3-48 | 2-Py | H | H | Me | Ph | Ph | $-(CH_2)_4-$ |
| 3-49 | 2-Py | H | H | Me | 4-FPh | 4-FPh | $-(CH_2)_2-$ |
| 3-50 | 2-Py | H | H | Et | Ph | Ph | $-(CH_2)_2-$ |
| 3-51 | 2-Py | H | H | Et | Ph | Ph | $-(CH_2)_3-$ |
| 3-52 | 3-Py | H | H | Et | Ph | Ph | $-(CH_2)_3-$ |
| 3-53 | 3-Py | H | H | Me | Ph | Ph | $-(CH_2)_2-$ |

Table 4

| Cpd. | $R^1$ | $R^2$ | $R^3$ | $R^4$ | $R^7$ | $R^8$ | A |
|---|---|---|---|---|---|---|---|
| 4-1 | 3-Py | H | H | H | Ph | Ph | $-(CH_2)_2-$ |
| 4-2 | 3-Py | H | H | H | Ph | Ph | $-(CH_2)_3-$ |
| 4-3 | 3-Py | H | H | H | Ph | Ph | $-(CH_2)_5-$ |
| 4-4 | 3-Py | H | H | H | 4-FPh | 4-FPh | $-(CH_2)_2-$ |
| 4-5 | 3-Py | H | H | H | 4-FPh | 4-FPh | $-(CH_2)_4-$ |
| 4-6 | 4-Py | H | H | H | 4-FPh | 4-FPh | $-(CH_2)_3-$ |
| 4-7 | 3-Py | H | H | H | Ph | 4-FPh | $-(CH_2)_3-$ |
| 4-8 | 3-Py | H | H | H | Ph | 4-ClPh | $-(CH_2)_5-$ |
| 4-9 | 3-Py | H | H | H | 4-ClPh | 4-ClPh | $-(CH_2)_3-$ |
| 4-10 | 3-Py | H | H | H | Ph | 4-MePh | $-(CH_2)_4-$ |
| 4-11 | 3-Py | H | H | H | Ph | 4-MeOPh | $-(CH_2)_2-$ |
| 4-12 | 4-Py | H | H | H | Ph | Ph | $-(CH_2)_3-$ |
| 4-13 | 3-Py | H | H | H | Ph | 3-FPh | $-(CH_2)_3-$ |
| 4-14 | 3-Py | H | H | H | 2-FPh | 4-ClPh | $-(CH_2)_3-$ |
| 4-15 | 3-Py | H | H | H | Ph | 3-ClPh | $-(CH_2)_3-$ |
| 4-16 | 3-Py | H | H | H | 4-ClPh | 2-ClPh | $-(CH_2)_3-$ |
| 4-17 | 3-Py | H | H | H | Ph | Ph | $-CH_2CH(Me)CH_2-$ |
| 4-18 | 3-Py | H | H | H | Ph | 4-FPh | $-CH_2CH(Me)CH_2-$ |
| 4-19 | 3-Py | H | H | H | Ph | 4-ClPh | $-CH_2CH(Me)CH_2-$ |
| 4-20 | 3-Py | H | H | H | 4-FPh | 4-FPh | $-CH_2CH(Me)CH_2-$ |
| 4-21 | 3-Py | H | Me | H | 4-FPh | 4-FPh | $-(CH_2)_2-$ |
| 4-22 | 3-Py | H | Me | H | 4-FPh | 4-FPh | $-(CH_2)_4-$ |
| 4-23 | 4-Py | H | H | H | Ph | Ph | $-(CH_2)_4-$ |
| 4-24 | 3-Py | H | H | Me | 4-FPh | 4-FPh | $-(CH_2)_2-$ |

Of these compounds, the following are preferred, that is to say Compounds No. 1-1, 1-2, 1-3, 1-4, 1-5, 1-6, 1-7, 1-12, 1-13, 1-14, 1-15, 1-16, 1-37, 1-54, 1-58, 1-64, 1-65, 1-68, 1-70, 1-71, 1-72, 1-75, 1-79, 1-83, 1-84, 1-86, 1-87, 1-88, 1-89, 1-90, 1-91, 2-12, 2-13, 2-14, 2-26, 2-32, 2-38, 2-40, 3-1, 3-2, 3-3, 3-4, 3-5, 3-6, 3-8, 3-19, 3-28, 3-30, 3-34, 3-35, 3-36, 3-38, 3-40, 3-41, 3-42, 3-43, 3-45, 3-46, 3-47, 3-48, 3-49, 3-52, 3-53, 4-1, 4-2, 4-4, 4-21 and 4-24, of which Compounds No. 1-12, 1-13, 1-65, 1-86, 1-87, 1-88, 1-89, 1-91, 2-12, 2-13, 2-32, 2-38, 3-3, 3-4, 3-5, 3-6, 3-34, 3-35, 3-36, 3-41, 3-47, 3-52, 3-53, 4-1, 4-2 and 4-4 are more preferred.

The most preferred compounds are Compounds No.:

1-12. {3-[4-bis(4-fluorophenyl)methyl-1-piperazinyl]propylcarbamoyl}-2-(3-pyridyl)thiazolidine;

1-65. {3-[4-(α-2-pyridylbenzyl)-1-piperazinyl]propylcarbamoyl}-2-(3-pyridyl)thiazolidine;

1-87. [2-(4-diphenylmethyl-1-piperazinyl)ethylcarbamoyl]-2-(3-pyridyl)thiazolidine;
1-88. [3-{4-[α-(4-chlorophenyl)benzyl]-1-piperazinyl}propylcarbamoyl]-2-(3-pyridyl)thiazolidine;
1-89. [2-{4-[α-(4-chlorophenyl)benzyl]-1-piperazinyl}ethylcarbamoyl]-2-(3-pyridyl)thiazolidine;
2-13. {3-[4-bis(4-fluorophenyl)methoxy-1-piperidyl]propylcarbamoyl}-2-(3-pyridyl)thiazolidine;
3-3. {2-[4-(diphenylmethylene)-1-piperidyl]ethylcarbamoyl}-2-(3-pyridyl)thiazolidine;
3-4. {3-[4-(diphenylmethylene)-1-piperidyl]propylcarbamoyl}-2-(3-pyridyl)thiazolidine;
3-5. {4-[4-(diphenylmethylene)-1-piperidyl]butylcarbamoyl}-2-(3-pyridyl)thiazolidine;
3-35. [3-{4-[bis(4-fluorophenyl)methylene]-1-piperidyl}propylcarbamoyl]-2-(3-pyridyl)thiazolidine;
3-41. {3-[4-(α-2-thienyl)benzylidene-1-piperidyl] propylcarbamoyl}-2-(3-pyridyl)thiazolidine;
3-52. [N-{3-[4-diphenylmethylene-1-piperidyl]propyl}-N-ethylcarbamoyl]-2-(3-pyridyl)thiazolidine;
3-53. [N-{2-[4-diphenylmethylene-1-piperidyl]ethyl}-N-methylcarbamoyl]-2-(3-pyridyl)thiazolidine; and
4-1. {2-[4-(α-hydroxydiphenylmethyl)-1-piperidyl] ethylcarbamoyl}-2-(3-pyridyl)thiazolidine;

and salts, especially hydrochlorides, thereof.

The compounds of the present invention may be prepared by a variety of methods well known in the art for the preparation of compounds of this type. For example, they may be prepared by reacting a compound of formula (VI):

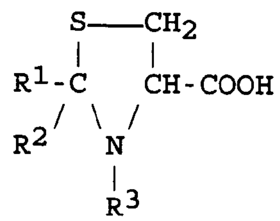

(VI)

(in which $R^1$, $R^2$ and $R^3$ are as defined above) or a reactive derivative thereof with a compound of formula (VII):

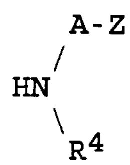

(VII)

(in which A, Z and $R^4$ are as defined above). Where either compound contains a reactive group in one of the substituents Z, $R^1$, $R^2$, $R^3$ and $R^4$, this may be protected prior to the reaction and the protecting group may then be removed after the reaction using methods well known in the art. If desired, the resulting compound of formula (I) may be salified.

The carboxylic acid of formula (VI) may be employed as such or a reactive derivative of the carboxylic acid of formula (VI) may be employed. Examples of suitable reactive derivatives include: acid halides, such as the acid chloride or acid bromide; the acid azide; active esters with, for example, N-hydroxybenzotriazole or N-hydroxysuccinimide; acid anhydrides of the carboxylic acid to be used; and mixed acid anhydrides with, for example, a mono($C_1$ - $C_4$ alkyl) carbonic acid ester, such as monomethyl carbonate, monoethyl carbonate or monoisobutyl carbonate, or a monoaryl carbonic acid ester, such as monophenyl carbonate or monotolyl carbonate, preferably a mixed acid anhydride with a monoalkyl carbonate.

The reaction between the carboxylic acid of formula (VI) itself and the amine of formula (VII) can be preferably carried out in the presence or absence of a base, in the presence of a condensing agent and in an inert solvent.

There is no particular restriction on the nature of the condensing agent to be used, provided that it can assist the formation of an amide bond from a carboxylic acid and an amine, and preferred examples include dicyclohexylcarbodiimide (DCC), diethylphosphoryl cyanide (DEPC), carbonyldiimidazole, diphenylphosphoryl azide (DPPA) and diethylazodicarboxylate/triphenylphosphine, more preferably dicyclohexylcarbodiimide or diethylphosphoryl cyanide.

There is likewise no particular limitation on the nature of the base to be used provided that it does not have any adverse effect on the reagents, and preferred examples include organic amines, such as trimethylamine, triethylamine, pyridine, dimethylaniline, N-methylmorpholine and N,N-dimethylpyridine, more preferably triethylamine or N-methylmorpholine.

There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene and xylene; halogenated hydrocarbons, especially halogenated aliphatic hydrocarbons, such as methylene chloride, dichloroethane and chloroform; esters, such as ethyl acetate and propyl acetate; ethers, such as diethyl ether, tetrahydrofuran and dioxane; amides, especially fatty acid amides, such as dimethylformamide, dimethylacetamide and hexamethylphosphoric triamide; and nitriles, such as acetonitrile. Of these, we most prefer the ethers (particularly tetrahydrofuran), the halogenated hydrocarbons (particularly methylene chloride), the amides (particularly dimethylformamide) and the esters (particularly ethyl acetate).

The reaction can take place over a wide range of temperatures, and the precise reaction temperature is not critical to the invention. In general, we find it convenient to carry out the reaction at a temperature of from -10 to 50°C, more preferably from 0 to 30°C. The time required for the reaction may also vary widely, depending on many factors, notably the reaction temperature and the nature of the reagents. However, provided that the reaction is effected under the preferred conditions outlined above, a period of from 30 minutes to 20 hours will usually suffice.

Alternatively, the desired compound of formula (I) can be prepared by converting the carboxylic acid of formula (VI) into a reactive derivative, and then reacting the reactive derivative with the amine of formula (VII).

The reactive derivative of the carboxylic acid, such as the acid halide or acid anhydride, can be prepared by conventional methods, for example by reacting the carboxylic acid of formula (VI) with a corresponding active halide (e.g. thionyl chloride, thionyl bromide, an acid chloride or acid bromide of the desired carboxylic acid to form a mixed anhydride, methyl chlorocarbonate, ethyl chlorocarbonate, isobutyl chlorocarbonate, phenyl chlorocarbonate or tolyl chlorocarbonate) at a suitable temperature, e.g. from 20 to 100°C, for a suitable time, e.g. from 1 to 20 hours, in an inert solvent (e.g. methylene chloride, benzene or tetrahydrofuran) and in the presence of a base (e.g. pyridine, triethylamine or dimethylaniline) as necessary. Where the reactive derivative is an acid amide or an active ester, this can be prepared by reacting the carboxylic acid of formula (VI) with the corresponding compound (e.g. hydrogen azide, N-hydroxybenzotriazole or N-hydroxysuccinimide); the conditions employed are similar to those employed to prepare an amide bond by reacting a carboxylic acid of formula (VI) with an amine of formula (VII) as described above.

The reaction of the reactive derivative of the carboxylic acid of formula (VI) with the amine of formula (VII) is preferably carried out in an inert solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride, dichloroethane and chloroform; ethers, such as diethyl ether, tetrahydrofuran and dioxane; esters, such as ethyl acetate; and aromatic hydrocarbons, such as benzene, toluene and xylene. Of these, we especially prefer the aromatic hydrocarbons and the ethers, such as tetrahydrofuran.

Alternatively, in some cases, the compound of formula (VII) can be used in a great excess, in which case it may serve also as a solvent.

The reaction can take place over a wide range of temperatures, and the precise reaction temperature is not critical to the invention. In general, we find it convenient to carry out the reaction at a temperature of from -10 to 50°C (more preferably from 0 to 25°C). The time required for the reaction may also vary widely, depending on many factors, notably the reaction temperature and the nature of the reagents. However, provided that the reaction is effected under the preferred conditions outlined above, a period of from 5 minutes to 20 hours (more preferably from 30 minutes to 10 hours) will usually suffice.

Further, the compound of formula (I) wherein $R^3$ represents a t-butoxycarbonyl group can, if desired, be reacted with an acid in an inert solvent to convert it into a corresponding compound where $R^3$ represents a hydrogen atom.

There is no particular restriction on the nature of the acid to be used here, and examples include: mineral acids, such as hydrochloric acid, hydrobromic acid, sulphuric acid and nitric acid; carboxylic acids, such as acetic acid, trifluoroacetic acid and benzoic acid; and sulphonic acids, such as methanesulphonic acid, benzenesulphonic acid and p-toluenesulphonic acid. Of these, we prefer hydrochloric acid, hydrobromic acid and trifluoroacetic acid.

There is likewise no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; ketones, such as acetone, methyl ethyl ketone and methyl isobutyl ketone; nitriles, such as acetonitrile; and water. Of these, we prefer the ethers and water.

The reaction can take place over a wide range of temperatures, and the precise reaction temperature is

not critical to the invention. In general, we find it convenient to carry out the reaction at a temperature of from 0 to 50°C (preferably around room temperature). The time required for the reaction may also vary widely, depending on many factors, notably the reaction temperature and the nature of the reagents. However, provided that the reaction is effected under the preferred conditions outlined above, a period of from 1 to 20 hours will usually suffice.

The compound prepared in the reactions described above can be collected from the reaction mixture by conventional means. For example, one suitable recovery procedure comprises neutralizing the reaction mixture and then evaporating the solvent from the neutralized mixture, or simply evaporating the solvent from the reaction mixture, as necessary; after this, the reaction mixture may be poured into water, and then extracted with a water-insoluble organic solvent; the desired compound can then be obtained by evaporation of the solvent from the extract, normally under reduced pressure. The product thus obtained can, if desired, be further purified by conventional methods, such as recrystallization, reprecipitation or the various chromatography techniques, notably column chromatography or preparative thin layer chromatography.

The starting compound of formula (VI) may be known per se or it may easily be prepared by known methods (e.g. FR 2 267 089; Japanese Kokai Hei 2-179) or similar methods.

The starting compound of formula (VII) may be known per se or it may easily be prepared by conventional methods [e.g. Chem. Pharm. Bull., 37, 100 (1989); J. Med. Chem., 32, 583 (1989)] or by similar methods. Alternatively, a compound of formula (VII) in which $R^4$ represents an alkyl group having from 1 to 4 carbon atoms can also be prepared by reacting the corresponding N-($C_1$ - $C_4$ aliphatic acyl) compounds {which can be prepared by conventional methods [e.g. J. Org. Chem., 27, 4058 (1962)] or by a similar reaction to that between the reactive derivative of the carboxylic acid of formula (VI), but using a derivative of the acid forming the aliphatic acyl group, and the compound of formula (VII)} with lithium aluminium hydride in a suitable solvent (e.g. an ether, such as diethyl ether or tetrahydrofuran) at from room temperature to 80°C for a period of from 30 minutes to 5 hours.

The thiazolidinecarboxylic acid amide derivatives of the present invention have excellent anti-allergic and anti-asthmatic activities, as well as PAF antagonism, and are thus useful as therapeutic agents for the treatment or prophylaxis of allergic diseases and asthma.

The compounds of the present invention may therefore be used in the treatment and prophylaxis of disorders such as those referred to above, and, for this purpose, may be formulated as conventional pharmaceutical preparations, as is well known in the art. Thus, the compounds may be administered orally, e.g. in the form of tablets, capsules, granules, powders, syrups, or other such well known forms, parenterally, e.g. by injections, suppositories, or by other means, for example, as patches, inhalation or ophthalmic solutions.

These pharmaceutical preparations can be prepared by conventional means and may contain known adjuvants of a type commonly used in this field, for example vehicles, binders, disintegrators, lubricants, stabilizers, corrigents, etc. depending upon the intended use and form of the preparation. The dose will depend upon the condition, age, and body weight of the patient as well as upon the nature and severity of the disorder to be treated, but, in the case of oral administration to an adult human patient, we would normally suggest a total daily dose of from 10 mg to 1000 mg, more preferably from 10 mg to 500 mg, which may be administered in a single dose or in divided doses, e.g. from one to three times a day.

## BIOLOGICAL ACTIVITY

The biological activity of the compounds of the present invention is shown in the following Experiments. In these Experiments, the compounds of the invention are identified by reference to the number of one of the subsequent Examples which illustrates their preparation.

### EXPERIMENT 1

#### Inhibitory effect on passive cutaneous anaphylaxis (PCA) in rats

According to Mota's method [I. Mota, Immunology, 7, 681 - 699 (1964)], antiserum (256 times the PCA titer) of rat against egg albumin was prepared and diluted four times with physiological saline. Male SD rats (5 weeks old) were used as the test animals in groups, each group containing 4 animals. The rats were sensitized by intradermal injection of 0.05 ml of the diluted antiserum solution in the dorsal position. 48 hours after this injection, a suspension of the test compound in an aqueous 0.5% w/v tragacanth solution was orally administered to the rats, which had been fasted for one day, and 60 minutes later they were injected in the caudal vein with 5 ml/kg body weight of physiological saline containing 0.4% w/v egg albumin and 1.0% w/v Evans Blue. 30 minutes after this last injection, the rats were sacrificed with carbon dioxide and the Evans Blue exuded in the

dorsal intradermal portion was determined according to Harada's method (Harada et al., J. Pharm. Pharmac., 23, 218 - 219 (1971)].

The results achieved from the test groups which were treated with a test compound were evaluated to determine the inhibitory rate by comparison with the average amount of exuded dye in a control group, which was not given the test compound.

The inhibitory rate was calculated by the following equation.

$$\text{Inhibitory rate (\%)} = (1-B/A) \times 100$$

A: amount of exuded dye in the control group

B: amount of exuded dye in the test group.

The results are shown in Table 5.

Table 5

| Compound of Example | Salt | Dose (p.o., mg/kg) | Inhibitory rate (%) |
|---|---|---|---|
| 14 | hydrochloride | 25 | 72 |
| | | 6.4 | 42 |
| 16 | hydrochloride | 25 | 65 |
| 17 | hydrochloride | 25 | 79 |
| | | 6.4 | 48 |
| 20 | free base | 25 | 76 |
| 22 | hydrochloride | 25 | 88 |
| | | 6.4 | 56 |
| 30 | free base | 6.4 | 57 |
| 44 | hydrochloride | 6.4 | 51 |

EXPERIMENT 2

Inhibitory effect in vitro against PAF-induced blood platelet aggregation

Blood samples were obtained by cardiac puncture from a rabbit and one part by volume of each sample was immediately mixed with 0.1 part of a 3.8% w/v aqueous solution of sodium citrate. A platelet rich plasma (PRP) fraction was prepared by centrifuging the samples at 150 x G for 15 minutes at room temperature, and a platelet poor plasma (PPP) fraction was then prepared by further centrifugation at 1,000 x G for 15 minutes. The platelet count in the PRP was adjusted to $6 \times 10^5$ per $\mu\ell$ by the addition of an appropriate amount of the PPP fraction. According to the method reported by Born et al. [G.V.R. Born et al.: J. Physiol. 62, 67 - 68 (1962)], blood platelet aggregation was determined turbidimetrically in a 6-channel aggregometer (Hemetracer, NKB,

Tokyo, Japan). Aliquots of the PRP (272 μℓ) were preincubated with 3 μℓ of a solution of the test compound in dimethyl sulphoxide for 1 minute, and then stimulated with ℓ-$C_{16:0}$ PAF (at a final concentration of $10^{-8}$ ~ 3 x $10^{-8}$ M) at 37°C with stirring (100 rpm). Changes in light transmission were monitored for 5 minutes. Vehicle (dimethyl sulphoxide) controls were tested simultaneously, and the inhibitory effects of the test compounds were assessed on the maximal aggregation. The $IC_{50}$ values were calculated by the method of least squares.

Table 6 below shows the results.

Table 6

| Compound of Example | Salt | Platelet aggregation inhibition, $IC_{50}$ (g/ml) |
|---|---|---|
| 17 | hydrochloride | 1.9 x $10^{-6}$ |
| 20 | free base | 3.3 x $10^{-6}$ |
| 22 | hydrochloride | 1.8 x $10^{-6}$ |
| 28 | hydrochloride | 6.0 x $10^{-7}$ |
| 38 | hydrochloride | 4.3 x $10^{-7}$ |
| 43 | hydrochloride | 9.5 x $10^{-7}$ |

## EXPERIMENT 3

### Inhibitory effect on PAF-receptor binding

Blood samples were drawn from the heart of a rabbit. 1 part by volume of each sample was mixed immediately with 1/9 part of a 0.077 M solution of disodium ethylenediaminetetraacetate. After a similar procedure to that described in Experiment 2, a precipitated blood platelet sample was obtained. This blood platelet sample was washed, and, after repeated freezing and thawing to rupture the cells, it was placed on top of two layers consisting of 0.25 M and 1.5 M sucrose solutions. By centrifugation at 63,500 x G, for 2 hours at 4°C, the fraction obtained from the interface between the 0.25 M and 1.5 M sucrose solutions was collected and is regarded as a PAF-receptor membrane fraction. A receptor binding experiment was then conducted according to a method very similar to that reported by Hwang et al. [San-Bao Hwang et al.: J. Biol. Chem. 260, 15639 - 15645 (1985)]. The specific binding of $^3$H-PAF was measured using a Wattman GF/C filter. A test compound was dissolved in dimethyl sulphoxide and diluted 100 fold with a buffer solution containing 0.5% bovine serum albumin. Nine parts by volume of the solution, for a receptor binding experiment, was mixed with one part of the test compound solution prepared above. The percent inhibition of the specific binding was plotted against the log of the concentration of the test compound, and the 50% inhibitory concentration ($IC_{50}$) was calculated from the linear line connecting all the plotted points.

The results are shown in Table 7.

Table 7

| Compound of Example | Salt | Receptor binding inhibition, $IC_{50}$ (g/ml) |
|---|---|---|
| 16 | hydrochloride | $3.8 \times 10^{-7}$ |
| 17 | hydrochloride | $6.8 \times 10^{-7}$ |
| 20 | free base | $6.3 \times 10^{-7}$ |
| 22 | hydrochloride | $6.7 \times 10^{-7}$ |
| 30 | hydrochloride | $2.5 \times 10^{-7}$ |
| 38 | hydrochloride | $3.8 \times 10^{-7}$ |
| 43 | hydrochloride | $5.4 \times 10^{-7}$ |

The invention is further illustrated by the following non-limiting Examples. Preparation of certain of the starting materials employed in these Examples is illustrated by the subsequent Preparations.

EXAMPLE 1

3-t-Butoxycarbonyl-4(R)-[3-(4-diphenylmethyl-1-piperazinyl)propylcarbamoyl]-2-(3-pyridyl)thiazolidine

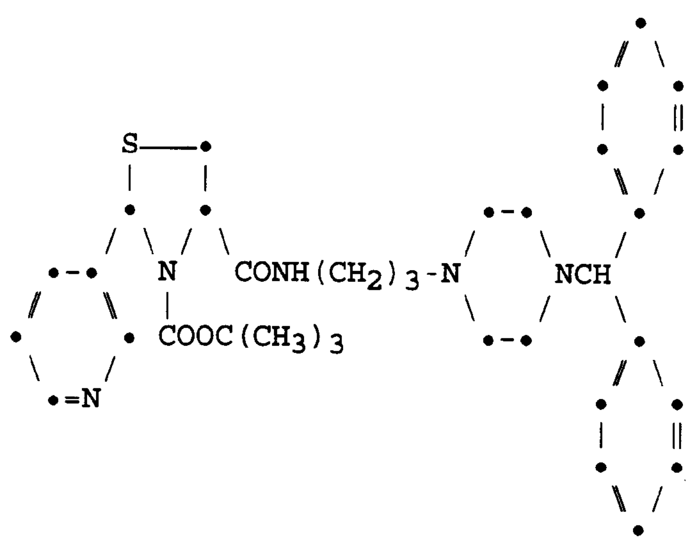

A mixture of 500 mg (1.61 mmole) of 3-t-butoxycarbonyl-2-(3-pyridyl)thiazolidine-4(R)-carboxylic acid, 500

mg (1.61 mmole) of 1-(3-aminopropyl)-4-(diphenylmethyl)piperazine (prepared as described in Preparation 16), 263 mg (1.61 mmole) of diethylphosphoryl cyanide, 163 mg (1.61 mmole) of triethylamine and 10 ml of tetrahydrofuran was stirred at room temperature overnight. At the end of this time, the reaction mixture was concentrated by evaporation under reduced pressure, diluted with water and extracted with methylene chloride. The solvent was then removed by evaporation under reduced pressure, and the resulting residue was subjected to column chromatography through alumina, using ethyl acetate as the eluent, to afford 840 mg (yield 89%) of the title compound as an oil.

Infrared Absorption Spectrum (KBr), $\nu_{max}$ $cm^{-1}$: 3350, 2973, 2934, 2808, 1669, 1367, 1158.

Mass Spectrum, m/z (%): 601 ($M^+$, 0.2), 407 (16), 167 (100).

EXAMPLE 2

4(R)-[3-(4-Diphenylmethyl-1-piperazinyl)propylcarbamoyl]-2-(3-pyridyl)thiazolidine and its hydrochloride

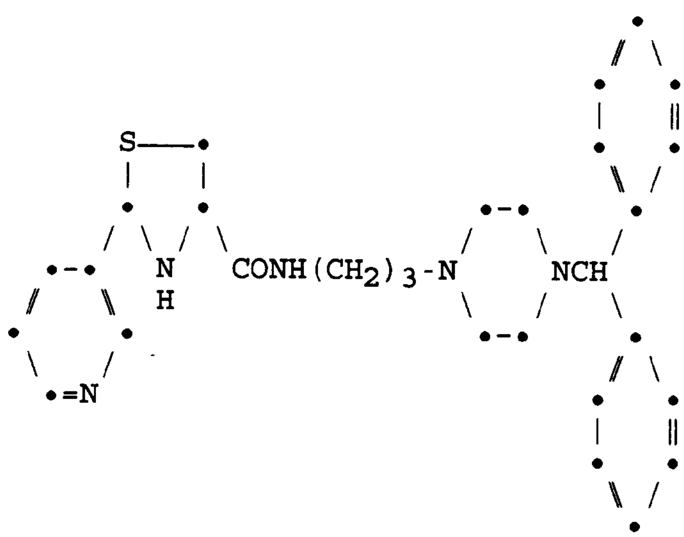

0.8 g (1.36 mmole) of 3-t-butoxycarbonyl-4(R)-[3-(4-diphenylmethyl-1-piperazinyl)propylcarbamoyl]-2-(3-pyridyl)thiazolidine (prepared as described in Example 1) were dissolved in 15 ml of 10% w/v aqueous hydrochloric acid, and the resulting solution was stirred at room temperature overnight. The reaction mixture was then neutralized and extracted with chloroform. The solvent was removed from the extract by evaporation under reduced pressure, and the resulting residue was subjected to column chromatography through silica gel, eluted with a 20 : 1 by volume mixture of methylene chloride and methanol, to afford 0.5 g (yield 74%) of the title compound as an oil.

Infrared Absorption Spectrum ($CHC\ell_3$), $\nu_{max}$ $cm^{-1}$: 3000, 2900, 2800, 1665, 1520, 1450.

Mass Spectrum, m/z (%): 501 ($M^+$, 4), 167 (100), 125 (48).

The oil thus obtained was dissolved in ethyl acetate, and a 4N solution of hydrogen chloride in ethyl acetate was added to the resulting solution. The crystals which precipitated were collected by filtration to afford the desired hydrochloride, melting at 193 to 195°C (with decomposition).

EXAMPLES 3 TO 10

A procedure similar to that described in Example 1 was repeated using the same carboxylic acid, except that the cyclic aminoalkylamine specified in each Example was used in place of the 4-(3-aminopropyl)-1-(diphenylmethyl)piperazine used in Example 1, to afford the following compounds.

EXAMPLE 3

3-t-Butoxycarbonyl-4(R)-[2-(4-diphenylmethyl-1-piperazinyl)ethylcarbamoyl]-2-(3-pyridyl)thiazolidine

This compound was obtained in a yield of 69%, from 4-(2-aminoethyl)-1-(diphenylmethyl)piperazine (a known compound).

Infrared Absorption Spectrum ($CHC\ell_3$), $\nu_{max}$ $cm^{-1}$: 3375, 2970, 1685, 1510.
Mass Spectrum, m/z (%): 587 ($M^+$, 4), 265 (48), 167 (100).

EXAMPLE 4

3-t-Butoxycarbonyl-4(R)-[4-(4-diphenylmethyl-1-piperazinyl)butylcarbamoyl]-2-(3-pyridyl)thiazolidine

This compound was obtained in a yield of 61%, from 1-(4-aminobutyl)-4-(diphenylmethyl)piperazine (prepared as described in Preparation 17).
Infrared Absorption Spectrum ($CHC\ell_3$), $\nu_{max}$ $cm^{-1}$: 3350, 2940, 1685, 1530.
Mass Spectrum, m/z (%): 615 ($M^+$, 5), 421 (72), 167 (100).

EXAMPLE 5

3-t-Butoxycarbonyl-4(R)-{2-[4-bis(4-fluorophenyl)methyl-1-piperazinyl]ethylcarbamoyl}-2-(3-pyridyl)thiazolidine

This compound was obtained in a yield of 73%, from 1-(2-aminoethyl)-4-[bis(4-fluorophenyl)methyl]piperazine (prepared as described in Preparation 18).
Infrared Absorption Spectrum ($CHC\ell_3$), $\nu_{max}$ $cm^{-1}$: 3340, 2975, 2937, 2814, 1698, 1505.
Mass Spectrum, m/z (%): 624 ($M^+$, 6), 393 (57), 203 (100).

EXAMPLE 6

3-t-Butoxycarbonyl-4(R)-{3-(4-bis(4-fluorophenyl)methyl-1-piperazinyl]propylcarbamoyl}-2-(3-pyridyl)thiazolidine

This compound was obtained in a yield of 87%, from 1-(3-aminopropyl)-4-[bis(4-fluorophenyl)methyl]piperazine (prepared as described in Preparation 19).
Infrared Absorption Spectrum ($CHC\ell_3$), $\nu_{max}$ $cm^{-1}$: 3350, 2973, 2938, 2811, 1698, 1505.
Mass Spectrum, m/z (%): 637 ($M^+$, 3), 407 (34), 203 (100).

EXAMPLE 7

3-t-Butoxycarbonyl-4(R)-{4-[4-bis(4-fluorophenyl)methyl-1-piperazinyl]butylcarbamoyl}-2-(3-pyridyl)thiazolidine

This compound was obtained in a yield of 70%, from 1-(4-aminobutyl)-4-[bis(4-fluorophenyl)methyl]piperazine (prepared as described in Preparation 20).
Infrared Absorption Spectrum ($CHC\ell_3$), $\nu_{max}$ $cm^{-1}$: 2970, 2930, 1685, 1505.
Mass Spectrum, m/z (%): 651 ($M^+$, 2), 421 (36), 203 (100).

EXAMPLE 8

3-t-Butoxycarbonyl-4(R)-[3-{4-(α-(4-chlorophenyl)benzyl]-1-piperazinyl}propylcarbamoyl]-2-(3-pyridyl)thiazolidine

This compound was obtained in a yield of 76%, from 1-(3-aminopropyl)-4-[α-(4-chlorophenyl)benzyl]piperazine (prepared as described in Preparation 23).
Infrared Absorption Spectrum ($CHC\ell_3$), $\nu_{max}$ $cm^{-1}$: 3350, 2970, 2830, 1690, 1525.
Mass Spectrum, m/z (%): 635 ($M^+$, 6), 407 (71), 201 (100).

EXAMPLE 9

3-t-Butoxycarbonyl-4(R)-[2-(4-diphenylmethylene-1-piperidyl)ethylcarbamoyl]-2-(3-pyridyl)thiazolidine

This compound was obtained in a yield of 70%, from 1-(2-aminoethyl)-4-(diphenylmethylene)piperidine (prepared as described in Preparation 24).

Infrared Absorption Spectrum ($CHCl_3$), $\nu_{max}$ $cm^{-1}$: 3330, 2974, 2930, 1700, 1544.
Mass Spectrum, m/z (%): 598 ($M^+$, 34), 323 (45), 262 (100).

EXAMPLE 10

3-t-Butoxycarbonyl-4(R)-[3-(4-diphenylmethylene-1-piperidyl)propylcarbamoyl]-2-(3-pyridyl)thiazolidine

This compound was obtained in a yield of 91%, from 1-(3-aminopropyl)-4-(diphenylmethylene)piperidine (prepared as described in Preparation 15).
Infrared Absorption Spectrum ($CHCl_3$), $\nu_{max}$ $cm^{-1}$: 3400, 2980, 1690, 1560.
Mass Spectrum, m/z (%): 584 ($M^+$, 18), 365 (12), 262 (100).

EXAMPLES 11 TO 16

The compounds obtained as described in Examples 3 to 8 were treated in the same manner as described in Example 2 to afford the compounds of Examples 11 to 16, respectively.

EXAMPLE 11

4(R)-[2-(4-Diphenylmethyl-1-piperazinyl)ethylcarbamoyl]-2-(3-pyridyl)thiazolidine

This compound, melting at 183 to 185°C, was obtained in a yield of 58%.
Infrared Absorption Spectrum (KBr), $\nu_{max}$ $cm^{-1}$: 3380, 3270, 1662, 1516.
Mass Spectrum, m/z (%): 487 ($M^+$, 19), 265 (53), 167 (100).

EXAMPLE 12

4(R)-[4-(4-Diphenylmethyl-1-piperazinyl)butylcarbamoyl]-2-(3-pyridyl)thiazolidine

This compound was obtained in a yield of 93%.
Infrared Absorption Spectrum ($CHCl_3$), $\nu_{max}$ $cm^{-1}$: 3380, 3270, 2950, 1670, 1520.
Mass Spectrum, m/z (%): 515 ($M^+$, 7), 481 (14), 321 (28), 167 (100).

EXAMPLE 13

4(R)-{2-[4-Bis(4-fluorophenyl)methyl-1-piperazinyl]ethylcarbamoyl}-2-(3-pyridyl)thiazolidine

This compound, melting at 162 to 164°C, was obtained in a yield of 92%.
Infrared Absorption Spectrum (KBr), $\nu_{max}$ $cm^{-1}$: 3380, 3263, 2808, 1658, 1506.
Mass Spectrum, m/z (%): 523 ($M^+$, 11), 301 (28), 203 (100).

EXAMPLE 14

4(R)-{3-[4-Bis(4-fluorophenyl)methyl-1-piperazinyl]propylcarbamoyl}-2-(3-pyridyl)thiazolidine and its hydrochloride

This compound was obtained in a yield of 92%.
Infrared Absorption Spectrum ($CHCl_3$), $\nu_{max}$ $cm^{-1}$: 3400, 2970, 1605, 1505.
Mass Spectrum, m/z (%): 537 ($M^+$, 19), 203 (100), 125 (41).
Treatment of the title compound with a 4N solution of hydrogen chloride in ethyl acetate, as described in Example 2, gave the hydrochloride, melting at 185 to 188°C (with decomposition).

EXAMPLE 15

4(R)-{4-[4-Bis(4-fluorophenyl)methyl-1-piperazinyl]butylcarbamoyl}-2-(3-pyridyl)thiazolidine and its hydrochloride

This compound was obtained in a yield of 86%.

Infrared Absorption Spectrum ($CHC\ell_3$), $\nu_{max}$ $cm^{-1}$: 3400, 2950, 1670, 1510.

Mass Spectrum, m/z (%): 551 ($M^+$, 3), 293 (10), 203 (100).

Treatment of the title compound with a 4N solution of hydrogen chloride in ethyl acetate, as described in Example 2, gave the hydrochloride, melting at 188 to 190°C (with decomposition).

EXAMPLE 16

4(R)-[3-{4-[α-(4-Chlorophenyl)benzyl]-1-piperazinyl}-propylcarbamoyl]-2-(3-pyridyl)thiazolidine and its hydrochloride

This compound was obtained in a yield of 75%.

Infrared Absorption Spectrum (KBr), $\nu_{max}$ $cm^{-1}$: 3290, 2940, 2810, 1666, 1520.

Mass Spectrum, m/z (%): 535 ($M^+$, 6), 201 (195), 165 (100).

Treatment of the title compound with a 4N solution of hydrogen chloride in ethyl acetate, as described in Example 2, gave the hydrochloride, melting at 188 to 190°C (with decomposition).

EXAMPLE 17

4(R)-{3-[4-Bis(4-fluorophenyl)methoxy-1-piperidyl]propylcarbamoyl}-2-(3-pyridyl)thiazolidine and its hydrochloride

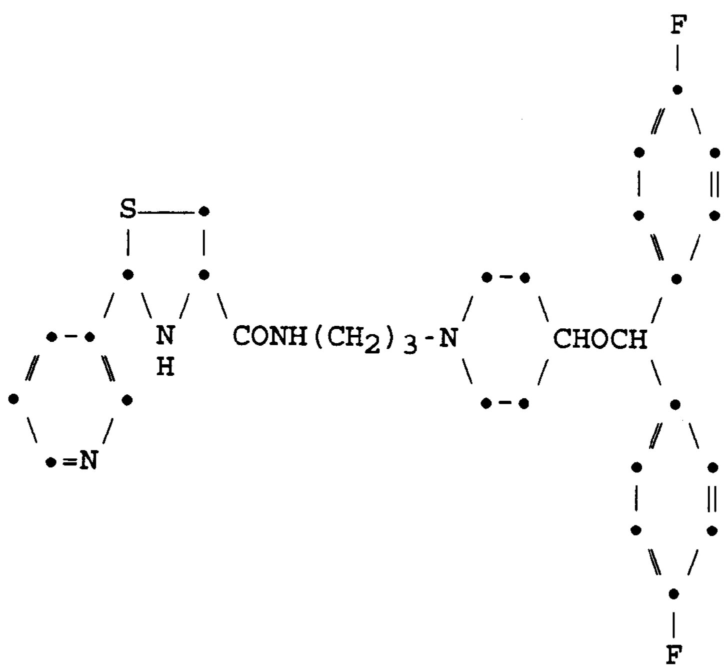

A mixture of 400 mg (1.90 mmole) of 2-(3-pyridyl)thiazolidine-4(R)-carboxylic acid, 686 mg (1.9 mmole) of-1-(3-aminopropyl)-4-[bis(4-fluorophenyl)methoxy]piperidine (prepared as described in Preparation 27), 392 mg (1.9 mmole) of dicyclohexylcarbodiimide, 257 mg (1.9 mmole) of 1-hydroxybenzotriazole and 6 ml of dimethylformamide was stirred overnight at room temperature. At the end of this time, the reaction mixture was diluted with ethyl acetate and insolubles were filtered off. A 0.5N aqueous solution of sodium hydroxide was added to the filtrate, and the mixture was extracted with ethyl acetate. A 1N aqueous solution of hydrochloric acid was added to the ethyl acetate extract, and the aqueous layer thus obtained was separated and made alkaline by the addition of a 2N aqueous solution of sodium hydroxide, after which it was extracted with ethyl acetate. The extract was concentrated by evaporation under reduced pressure, and the resulting residue was subjected to column chromatography through alumina, using a 20 : 1 by volume mixture of ethyl acetate and ethanol as the eluent, to afford 710 mg (yield 68%) of the title compound as an oil.

Infrared Absorption Spectrum ($CHC\ell_3$), $\nu_{max}$ $cm^{-1}$: 3400, 3300, 2950, 1665, 1605, 1505.

Mass Spectrum, m/z (%): 552 ($M^+$, 1), 384 (12), 165 (100).

The oil obtained was dissolved in ethyl acetate and treatment of the resulting solution with a 4N solution

of hydrogen chloride in ethyl acetate, as described in Example 2, gave the hydrochloride, melting at 114 to 117°C (with decomposition).

EXAMPLES 18 & 19

Following a procedure similar to that described in Example 17, but using the piperidinoalkylamine specified, the compounds shown were also obtained.

The hydrochlorides of these compounds were also obtained in quantitative yields by following the procedure described in Example 2.

EXAMPLE 18

4(R)-{2-[4-Bis(4-fluorophenyl)methoxy-1-piperidyl]ethylcarbamoyl}-2-(3-pyridyl)thiazolidine

The title compound was obtained in a yield of 55% from 1-(2-aminoethyl)-4-[bis(4-fluorophenyl)methoxy]piperidine (prepared as described in Preparation 26).

Infrared Absorption Spectrum ($CHCl_3$), $\nu_{max}$ $cm^{-1}$: 3350, 2930, 1665, 1510.

Mass Spectrum, m/z (%): 538 ($M^+$, 0.3), 316 (39), 203 (100).

The hydrochloride was then obtained as a hygroscopic powder, melting at 75 to 77°C.

EXAMPLE 19

4(R)-{3-[4-(α-Hydroxydiphenylmethyl)-1-piperidyl]propylcarbamoyl}-2-(3-pyridyl)thiazolidine

The title compound was obtained in a yield of 65% from 1-(3-aminopropyl)-4-(α-hydroxydiphenylmethyl)piperidine (prepared as described in Preparation 28).

Infrared Absorption Spectrum ($CHCl_3$), $\nu_{max}$ $cm^{-1}$: 3289, 2942, 1660, 1524.

Mass Spectrum, m/z (%): 516 ($M^+$, 1), 280 (69), 28 (100).

The hydrochloride was then obtained, melting at 110 to 113°C.

EXAMPLE 20

4(R)-[2-{4-[α-(4-Chlorophenyl)benzyl]-1-piperazinyl}-ethylcarbamoyl]-2-(3-pyridyl)thiazolidine

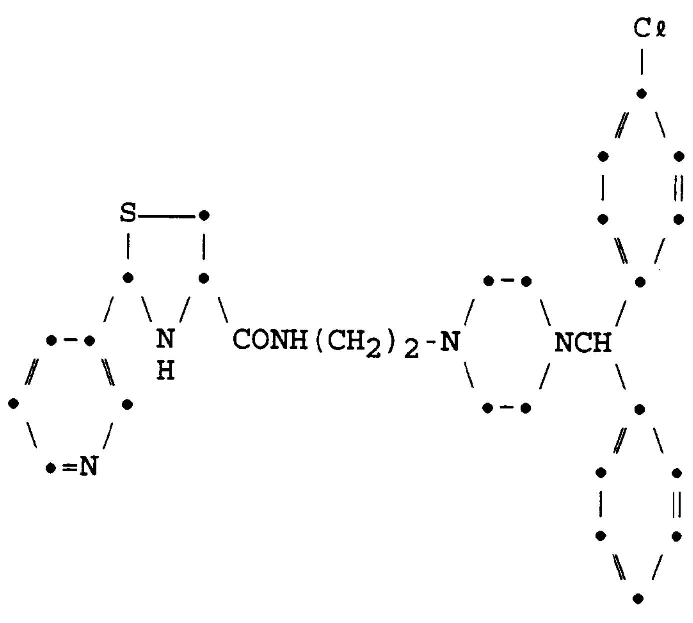

A mixture of 408 mg (1.94 mmole) of 2-(3-pyridyl)thiazolidine-4(R)-carboxylic acid, 640 mg (1.94 mmole) of 1-(2-aminoethyl)-4-[α-(4-chlorophenyl)benzyl]piperazine (prepared as described in Preparation 22), 400 mg (1.94 mmole) of dicyclohexylcarbodiimide, 262 mg (1.94 mmole) of 1-hydroxybenzotriazole and 12 ml of

dimethylformamide was stirred overnight at room temperature. At the end of this time, the reaction mixture was diluted with ethyl acetate and insolubles were filtered off. A 5% w/v aqueous solution of sodium hydrogencarbonate was added to the filtrate, and the ethyl acetate layer was separated and concentrated by evaporation under reduced pressure. The resulting residue was subjected to column chromatography through alumina, using ethyl acetate as the eluent, to afford 580 mg (yield 58%) of the title compound as crystals, melting at 157 - 159°C (after recrystallization from ethanol).

Mass Spectrum, m/z (%): 521 ($M^+$, 13), 295 (51), 201 (100).

EXAMPLE 21

4(R)-{2-[4-(Diphenylmethylene)-1-piperidyl]ethylcarbamoyl}-2-(3-pyridyl)thiazolidine and its hydrochloride

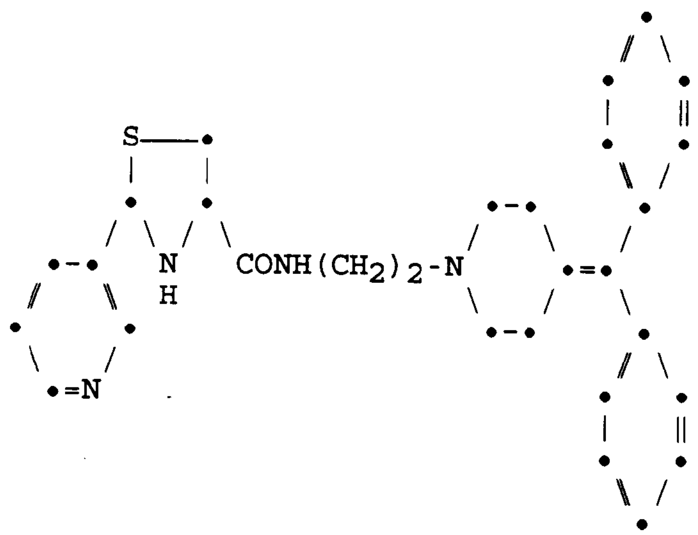

A mixture of 1.1 g (1.88 mmole) of 3-t-butoxycarbonyl-4(R)-[2-(4-diphenylmethylene-1-piperidyl)ethylcarbamoyl]-2-(3-pyridyl)thiazolidine (prepared as described in Example 9), 10 ml of a 4N solution of hydrogen chloride in dioxane and 10 ml of methylene chloride was stirred at room temperature for 3 hours. At the end of this time, the reaction mixture was poured into ice water, neutralized by the addition of a 2N aqueous solution of sodium hydroxide and extracted with chloroform. The extract was concentrated by distillation under reduced pressure, and the resulting residue was subjected to column chromatography through silica gel, using a 10 : 2 by volume mixture of chloroform and methanol as the eluent, to afford 1.0 g (a quantitative yield) of the title compound as an oil.

Infrared Absorption Spectrum ($CHCl_3$), $\nu_{max}$ $cm^{-1}$: 3250, 2982, 1677, 1265.

Mass Spectrum, m/z (%): 484 ($M^+$, 13), 262 (100).

The oil obtained was dissolved in ethyl acetate and a 4N solution of hydrogen chloride in ethyl acetate was added to the resulting solution. The crystals which precipitated were collected by filtration to afford the hydrochloride of the title compound, melting at 102 to 105°C.

EXAMPLE 22

4(R)-{3-[4-(Diphenylmethylene)-1-piperidyl]propylcarbamoyl}-2-(3-pyridyl)thiazolidine and its hydrochloride

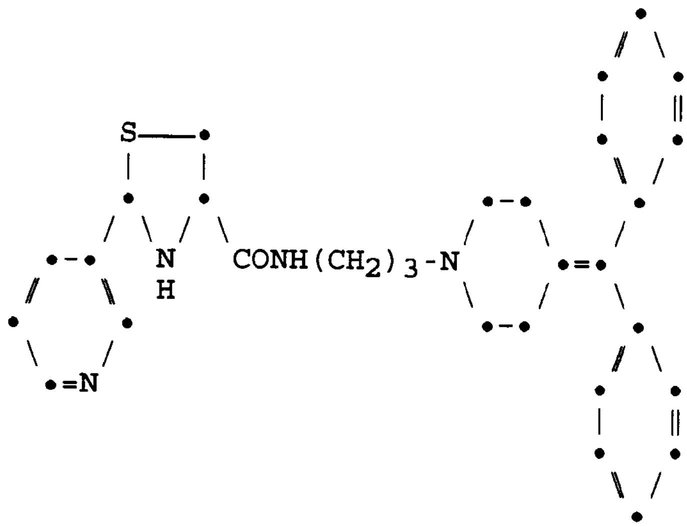

The title compound was obtained in a yield of 68% by following the procedure described in Example 20 and using the same carboxylic acid as was used in Example 20 and 1-(3-aminopropyl)-4-(diphenylmethylene)piperidine (prepared as described in Preparation 15).

Infrared Absorption Spectrum (KBr), $\nu_{max}$ $cm^{-1}$: 3300, 2942, 1979, 1661.

Mass Spectrum, m/z (%): 498 ($M^+$, 14), 359 (24), 262 (100).

The hydrochloride of the title compound, melting at 140 - 142°C (with decomposition), was obtained in the same manner as described in Example 2.

EXAMPLE 23

4(R)-{4-[4-(Diphenylmethylene)-1-piperidyl]butylcarbamoyl}-2-(3-pyridyl)thiazolidine and its hydrochloride

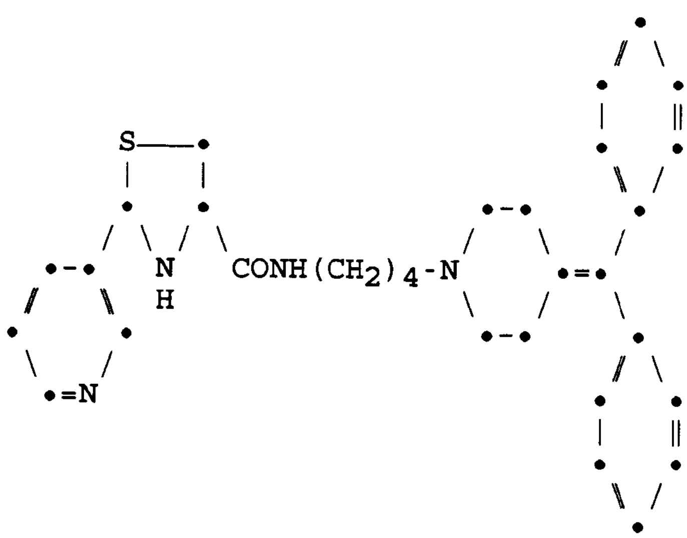

The title compound was obtained in a yield of 46% by following the procedure described in Example 20 and using the same carboxylic acid as was used in Example 20 and 1-(4-aminobutyl)-4-(diphenylmethylene)piperidine (prepared as described in Preparation 25).

Infrared Absorption Spectrum ($CHC\ell_3$), $\nu_{max}$ $cm^{-1}$: 3400, 3050, 2960, 1735, 1665, 1530.

Mass Spectrum, m/z (%): 512 ($M^+$, 1), 262 (100).

The hydrochloride of the title compound, melting at 122 to 124°C, was obtained in the same manner as described in Example 2.

EXAMPLE 24

4(R)-[5-{4-[Bis(4-fluorophenyl)methyl]-1-piperazinyl}pentylcarbamoyl]-2-(3-pyridinyl)thiazolidine and its hydrochloride

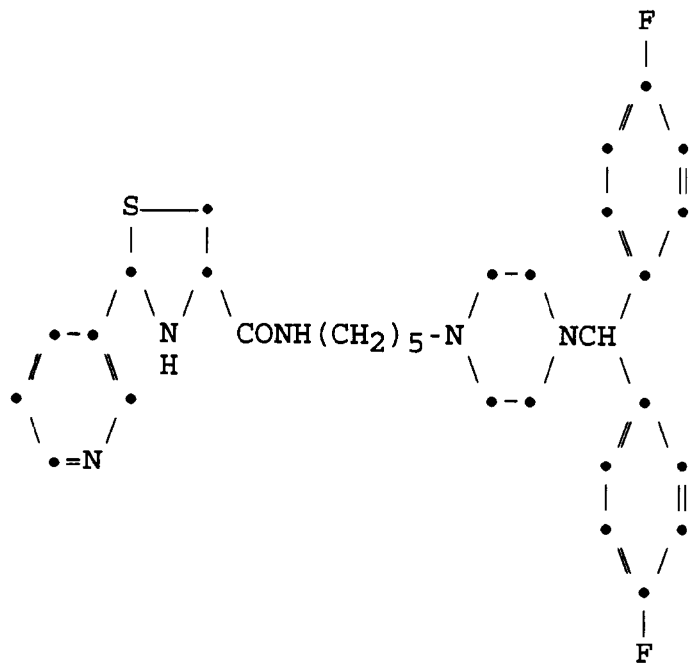

A mixture of 500 mg (2.4 mmole) of 2-(3-pyridyl)thiazolidine-4(R)-carboxylic acid, 900 mg (2.4 mmole) of 1-(5-aminopentyl)-4-[bis(4-fluorophenyl)methyl]piperazine (prepared as described in Preparation 21), 497 mg (2.4 mmole) of dicyclohexylcarbodiimide, 326 mg (2.4 mmole) of 1-hydroxybenzotriazole and 8 ml of dimethylformamide was stirred overnight at room temperature. At the end of this time, the reaction mixture was diluted with ethyl acetate and insolubles were filtered off. A 0.5N aqueous solution of sodium hydroxide was added to the filtrate, after which the mixture was extracted with ethyl acetate. A 1N aqueous solution of hydrochloric acid was added to the ethyl acetate extract, and the aqueous layer thus obtained was separated and made alkaline by the addition of a 2N aqueous solution of sodium hydroxide. It was then extracted with ethyl acetate. The extract was concentrated by evaporation under reduced pressure, and the resulting residue was subjected to column chromatography through silica gel, using a 20 : 1 by volume mixture of chloroform and methanol as the eluent, to afford 800 mg (yield 60%) of the title compound as an oil.

Infrared Absorption Spectrum ($CHC\ell_3$), $\nu_{max}$ $cm^{-1}$: 3400, 2960, 2830, 1745, 1670, 1610, 1505.

Mass Spectrum, m/z (%): 565 ($M^+$, 0.3), 203 (100).

The oil thus obtained was dissolved in ethyl acetate and a 4N solution of hydrogen chloride in ethyl acetate was added to the resulting solution, to afford the hydrochloride of the title compound, melting at 174 to 176°C (with decomposition).

EXAMPLE 25

4(R)-[2-{4-[Bis(4-fluorophenyl)-α-hydroxymethyl]-1-piperidyl}ethylcarbamoyl]-2-(3-pyridyl)thiazolidine and its hydrochloride

The title compound was prepared in a yield of 42% in a similar manner to that described in Example 17 by reacting 2-(3-pyridyl)thiazolidine-4(R)-carboxylic acid and 1-(2-aminoethyl)-4-[bis(4-fluorophenyl)-α-hydroxymethyl]piperidine (prepared as described in Preparation 55).

Infrared Absorption Spectrum ($CHC\ell_3$), $\nu_{max}$ $cm^{-1}$: 3350, 2935, 1660, 1505.

Mass spectrum, m/z (%): 538 ($M^+$, 1), 316 (100), 298 (19).

The hydrochloride of the title compound, melting at 143 - 145°C, was prepared in a similar manner to that described in Example 2.

EXAMPLE 26

4(R)-[2-{4-[Bis(4-fluorophenyl)methylene]-1-piperidyl}ethylcarbamoyl]-2-(3-pyridyl)thiazolidine

The title compound, melting at 119 - 121°C, was prepared in a yield of 91% in a similar manner to that described in Example 17 by reacting 2-(3-pyridyl)thiazolidine-4(R)-carboxylic acid and 1-(2-aminoethyl)4-[bis(4-fluorophenyl)methylene]piperidine (prepared as described in Preparation 56).

Infrared Absorption Spectrum ($CHC\ell_3$), $\nu_{max}$ $cm^{-1}$: 3400, 2960, 1675, 1510.

Mass spectrum, m/z (%): 520 ($M^+$, 8), 298 (100)

EXAMPLE 27

4(R)-[5-(4-Diphenylmethylene-1-piperidyl)pentylcarbamoyl]-2-(3-pyridyl)thiazolidine and its hydrochloride

The title compound was prepared in a yield of 36% in a similar manner to that described in Example 17 by reacting 2-(3-pyridyl)thiazolidine-4(R)-carboxylic acid and 1-(5-aminopentyl)-4-(diphenylmethylene)piperidine (prepared as described in Preparation 42).

Infrared Absorption Spectrum ($CHC\ell_3$), $\nu_{max}$ $cm^{-1}$: 2950, 1670, 1530, 1450.

Mass spectrum, m/z (%): 526 ($M^+$, 4), 262 (100).

The hydrochloride of the title compound, melting at 74 - 77°C, was prepared in a similar manner to that described in Example 2.

EXAMPLE 28

4(R)-[7-(4-Diphenylmethylene-1-piperidyl)heptylcarbamoyl]-2-(3-pyridyl)thiazolidine

The title compound was prepared in a yield of 46% in a similar manner to that described in Example 17 by reacting 2-(3-pyridyl)thiazolidine-4(R)-carboxylic acid and 1-(7-aminoheptyl)-4-(diphenylmethylene)piperidine (prepared as described in Preparation 43).

Infrared Absorption Spectrum ($CHC\ell_3$), $\nu_{max}$ $cm^{-1}$: 3400, 2935, 1670, 1525.

Mass spectrum, m/z (%): 554 ($M^+$, 0.3), 262 (100).

EXAMPLE 29

4(R)-[3-{4-[Bis(4-fluorophenyl)methylene]-1-piperidyl}propylcarbamoyl]-2-(3-pyridyl)thiazolidine and its hydrochloride

The title compound was prepared in a yield of 15% in a similar manner to that described in Example 17 by reacting 2-(3-pyridyl)thiazolidine-4(R)-carboxylic acid and 1-(3-aminopropyl)-4-[bis(4-fluorophenyl)methylene]piperidine (prepared as described in Preparation 57).

Infrared Absorption Spectrum ($CHC\ell_3$), $\nu_{max}$ $cm^{-1}$: 3375, 3275, 2935, 2800, 1665, 1600, 1505.

Mass spectrum, m/z (%): 534 ($M^+$, 18), 395 (31), 298 (100).

The hydrochloride of the title compound, melting at 95 - 98°C (with decomposition), was prepared in a similar manner to that described in Example 2.

EXAMPLE 30

4(R)-{3-[4-(α-2-Pyridylbenzyl)-1-piperazinyl]propylcarbamoyl}-2-(3-pyridyl)thiazolidine

The title compound was prepared in a yield of 16% in a similar manner to that described in Example 17 by reacting 2-(3-pyridyl)thiazolidine-4(R)-carboxylic acid and 4-[α-(2-pyridyl)benzyl]-1-(3-aminopropyl)piperazine (prepared as described in Preparation 41).

Infrared Absorption Spectrum ($CHC\ell_3$), $\nu_{max}$ $cm^{-1}$: 3300, 2960, 2830, 1670, 1590, 1520.

Mass spectrum, m/z (%): 502 ($M^+$, 6), 197 (52), 169 (100).

EXAMPLE 31

4(R)-{2-[4-(α-3-Pyridylbenzyl)-1-piperazinyl]ethylcarbamoyl}-2-(3-pyridyl)thiazolidine

The title compound, melting at 130 - 131°C, was prepared in a yield of 56% in a similar manner to that described in Example 17 by reacting 2-(3-pyridyl)thiazolidine-4(R)-carboxylic acid and 1-(2-aminoethyl)-4-[α-(3-pyridyl)benzyl]piperazine (prepared as described in Preparation 58).

Infrared Absorption Spectrum ($CHC\ell_3$), $\nu_{max}$ $cm^{-1}$: 3380, 3010, 2970, 2830, 1670, 1580, 1520.

Mass spectrum, m/z (%): 486 ($M^+$-2, 30), 266 (52), 168 (100).

EXAMPLE 32

4(R)-{2-[4-(α-4-Pyridylbenzyl)-1-piperazinyl]ethylcarbamoyl}-2-(3-pyridyl)thiazolidine

The title compound, melting at 160 - 162°C, was prepared in a yield of 60% in a similar manner to that described in Example 17 by reacting 2-(3-pyridyl)thiazolidine-4(R)-carboxylic acid and 1-(2-aminoethyl)-4-[α-(4-pyridyl)benzyl]piperazine (prepared as described in Preparation 59).

Infrared Absorption Spectrum ($CHC\ell_3$), $\nu_{max}$ $cm^{-1}$: 3400, 3000, 2950, 2830, 1670, 1600, 1520.

Mass spectrum, m/z (%): 488 ($M^+$, 13), 266 (100), 168 (98).

EXAMPLE 33

4(R)-{2-[4-(α-2-Pyridyl-4-fluorobenzyl)-1-piperaziny]ethylcarbamoy}-2-(3-pyridyl)thiazolidine

The title compound, melting at 144 - 146°C, was prepared in a yield of 71% in a similar manner to that described in Example 17 by reacting 2-(3-pyridyl)thiazolidine-4(R)-carboxylic acid and 1-(2-aminoethyl)-4-[α-(2-pyridyl)-4-fluorobenzyl]piperazine (prepared as described in Preparation 60).

Infrared Absorption Spectrum ($CHC\ell_3$), $\nu_{max}$ $cm^{-1}$: 3380, 3000, 2950, 2820, 1670, 1605, 1590, 1505.

Mass spectrum, m/z (%): 506 ($M^+$, 10), 215 (46), 186 (100).

EXAMPLE 34

4(R)-{2-[4-(α-2-Pyridyl-4-trifluoromethylbenzyl)-1-piperazinyl]ethylcarbamoyl}-2-(3-pyridyl)thiazolidine and its hydrochloride

The title compound was prepared in a yield of 54% in a similar manner to that described in Example 17 by reacting 2-(3-pyridyl)thiazolidine-4(R)-carboxylic acid and 1-(2-aminoethyl)-4-[α-(2-pyridyl)-4-trifluoromethylbenzyl]piperazine (prepared as described in Preparation 61).

Infrared Absorption Spectrum ($CHC\ell_3$), $\nu_{max}$ $cm^{-1}$: 3380, 2960, 2825, 1670, 1590, 1520.

Mass Spectrum, m/z (%): 556 ($M^+$, 18), 265 (71), 236 (100).

The hydrochloride of the title compound, melting at 164 - 167°C (with decomposition), was prepared in a similar manner to that described in Example 2.

EXAMPLE 35

4(R)-{2-[4-Bis(4-fluorophenyl)methylene-1-piperidyl]ethylcarbamoyl}-3-methyl-2-(3-pyridyl)thiazolidine and its hydrochloride

The title compound was prepared in a yield of 82% in a similar manner to that described in Example 17 by reacting 3-methyl-2-(3-pyridyl)thiazolidine-4(R)-carboxylic acid and 1-(2-aminoethyl)-[4-bis(4-fluorophenyl)methylene]piperidine (prepared as described in Preparation 56).

Infrared Absorption Spectrum ($CHC\ell_3$), $\nu_{max}$ $cm^{-1}$: 3380, 3120, 3060, 2820, 1670, 1605, 1510.

Mass spectrum, m/z (%): 534 ($M^+$, 18), 298 (100), 179 (29).

The hydrochloride of the title compound, melting at 148 - 150°C (with decomposition), was prepared in a similar manner to that described in Example 2.

EXAMPLE 36

4(R)-{2-[4-Bis(4-fluorophenyl)-α-hydroxymethyl-1-piperidyl]ethylcarbamoyl}-3- methyl-2-(3-pyridyl)thiazolidine and its hydrochloride

The title compound was prepared in a yield of 57% in a similar manner to that described in Example 17 by reacting 3-methyl-2-(3-pyridyl)thiazolidine-4(R)-carboxylic acid and 1-(2-aminoethyl)-[4-bis(4-fluorophenyl)-α-hydroxymethyl]piperidine (prepared as described in Preparation 55).

Infrared Absorption Spectrum ($CHC\ell_3$), $\nu_{max}$ $cm^{-1}$: 3360, 2980, 2940, 1665, 1600, 1505.

Mass spectrum, m/z (%): 520 ($M^+$, 3), 316 (100), 123 (22).

The hydrochloride of the title compound, melting at 115 - 117°C (with decomposition), was prepared in a similar manner to that described in Example 2.

EXAMPLE 37

4(R)-{2-[4-(α-2-Pyridyl)benzyl-1-piperazinyl]ethylcarbamoyl}-3-methyl-2-(3-pyridyl)thiazolidine and its hydrochloride

The title compound was prepared in a yield of 93% in a similar manner to that described in Example 17 by reacting 3-methyl-2-(3-pyridyl)thiazolidine-4(R)-carboxylic acid and 1-(2-aminoethyl)-[4-(α-2-pyridyl)benzyl]piperazine (prepared as described in Preparation 62).

Infrared Absorption Spectrum ($CHC\ell_3$), $\nu_{max}$ $cm^{-1}$: 3360, 2980, 2940, 1665, 1600, 1505.

Mass spectrum, m/z (%): 502 ($M^+$, 27), 334 (73), 169 (100).

The hydrochloride of the title compound, melting at 148 - 150°C, was prepared in a similar manner to that described in Example 2.

EXAMPLE 38

4(R)-{3-[4-(α-2-Thienyl)benzylidene-1-piperidyl]propylcarbamoyl}-2-(3-pyridyl)thiazolidine and its hydrochloride

The title compound was prepared in a yield of 47% in a similar manner to that described in Example 17 by reacting 2-(3-pyridyl)thiazolidine-4(R)-carboxylic acid and 1-(3-aminopropyl)-4-[α-(2-thienyl)benzylidene]piperidine (prepared as described in Preparation 63).

Infrared Absorption Spectrum ($CHC\ell_3$), $\nu_{max}$ $cm^{-1}$: 3300, 3000, 2930, 1665, 1520.

Mass spectrum, m/z (%): 504 ($M^+$, 19), 268 (100), 129 (37).

The hydrochloride of the title compound, melting at 133 - 135°C (with decomposition), was prepared in a similar manner to that described in Example 2.

EXAMPLE 39

4(R)-{N-[2-(4-Diphenylmethyl-1-piperazinyl)ethyl]N-methylcarbamoyl}-2-(3-pyridyl)thiazolidine and its hydrochloride

The title compound was prepared in a yield of 46% in a similar manner to that described in Example 17 by reacting 2-(3-pyridyl)thiazolidine-4(R)-carboxylic acid and 4-diphenylmethyl-1-[2-(N-methylamino)ethyl]piperazine (prepared as described in Preparation 36).

Infrared Absorption Spectrum ($CHC\ell_3$), $\nu_{max}$ $cm^{-1}$: 3320, 3020, 2960, 1650, 1495.

Mass spectrum, m/z (%): 501 ($M^+$, 4), 265 (39), 167 (100).

The hydrochloride of the title compound, melting at 185 - 188°C (with decomposition), was prepared in a similar manner to that described in Example 2.

EXAMPLE 40

4(R)-{2-[4-Bis(4-fluorophenyl)methylene-1-piperidyl]ethylcarbamoyl}-2-[5-(2-methylpyridyl)]thiazolidine and its hydrochloride

The title compound was prepared in a yield of 50% in a similar manner to that described in Example 17 by

reacting 2-[5-(2-methylpyridyl)]thiazolidine-4(R)-carboxylic acid and 1-(2-aminoethyl)-4-[bis(4-fluorophenyl)methylene]piperidine (prepared as described in Preparation 56).

Infrared Absorption Spectrum ($CHC\ell_3$), $\nu_{max}$ $cm^{-1}$: 3200, 2950, 1670, 1605, 1510, 1220.

Mass spectrum, m/z (%): 534 ($M^+$, 10), 298 (100).

The hydrochloride of the title compound, melting at 144 - 146°C (with decomposition), was prepared in a similar manner to that described in Example 2.

## EXAMPLE 41

### 4(R)-{3-[4-Bis(4-fluorophenyl)methylene-1-piperidyl]propylcarbamoyl}-2-[5-(2-methylpyridyl)]thiazolidine and its hydrochloride

The title compound was prepared in a yield of 33% in a similar manner to that described in Example 17 by reacting 2-[5-(2-methylpyridyl)]thiazolidine-4(R)-carboxylic acid and 1-(3-aminopropyl)-4-[bis(4-fluorophenyl)methylene]piperidine (prepared as described in Preparation 57).

Infrared Absorption Spectrum ($CHC\ell_3$), $\nu_{max}$ $cm^{-1}$: 3300, 2950, 1670, 1605, 1510, 1220.

Mass spectrum, m/z (%): 548 ($M^+$, 24), 395 (40), 298 (100).

The hydrochloride of the title compound, melting at 146 - 148°C, was prepared in a similar manner to that described in Example 2.

## EXAMPLE 42

### 4(R)-{3-[4-Diphenylmethylene-1-piperidyl]propylcarbamoyl}-2-[5-(2-methylpyridyl)]thiazolidine and its hydrochloride

The title compound was prepared in a yield of 80% in a similar manner to that described in Example 17 by reacting 2-[5-(2-methylpyridyl)]thiazolidine-4(R)-carboxylic acid and 1-(3-aminopropyl)-4-(diphenylmethylene)piperidine (prepared as described in Preparation 15).

Infrared Absorption Spectrum ($CHC\ell_3$), $\nu_{max}$ $cm^{-1}$: 3300, 2950, 1670, 1605, 1525, 1495.

Mass spectrum, m/z (%): 512 ($M^+$, 41), 359 (53), 212 (100).

The hydrochloride of the title compound, melting at 98 - 101°C, was prepared in a similar manner to that described in Example 2.

## EXAMPLE 43

### 4(R)-[N-{3-[4-Diphenylmethylene-1-piperidyl]propyl}-N-ethylcarbamoyl]-2-(3-pyridyl)thiazolidine and its hydrochloride

The title compound was prepared in a yield of 27% in a similar manner to that described in Example 17 by reacting 2-(3-pyridyl)thiazolidine-4(R)-carboxylic acid and 4-diphenylmethylene-1-[3-(N-ethylamino)propyl]piperidine (prepared as described in Preparation 32).

Infrared Absorption Spectrum ($CHC\ell_3$), $\nu_{max}$ $cm^{-1}$: 3000, 1680, 1640, 1380.

Mass spectrum, m/z (%): 526 ($M^+$, 47), 387 (52), 262 (100).

The hydrochloride of the title compound, melting at 92 - 94°C, was prepared in a similar manner to that described in Example 2.

## EXAMPLE 44

### 4(R)-[N-{2-[4-Diphenylmethylene-1-piperidyl]ethyl}-N-methylcarbamoyl]-2-(3-pyridyl)thiazolidine and its hydrochloride

The title compound was prepared in a yield of 82% in a similar manner to that described in Example 17 by reacting 2-(3-pyridyl)thiazolidine-4(R)-carboxylic acid and 4-diphenylmethylene-1-[2-(N-methylamino)ethyl]-piperidine (prepared as described in Preparation 30).

Infrared Absorption Spectrum ($CHC\ell_3$), $\nu_{max}$ $cm^{-1}$: 3000, 1740, 1640, 1500, 1400.

Mass spectrum, m/z (%): 498 ($M^+$, 8), 262 (100).

The hydrochloride of the title compound, melting at 135 - 140°C, was prepared in a similar manner to that described in Example 2.

Example 45

4(S)-{3-(4-Diphenylmethylene-1-piperidyl]-propylcarbamoyl}-2-(3-pyridyl)thiazolidine and its hydrochloride

The title compound was prepared in a yield of 27% in a similar manner to that described in Example 17 by reacting 2-(3-pyridyl)thiazolidine-4(S)-carboxylic acid (prepared as described in Preparation 29) and 1-(3-aminopropyl)-4-(diphenylmethylene)piperidine (prepared as described in Preparation 15).

Infrared Absorption Spectrum ($CHC\ell_3$), $\nu_{max}$ $cm^{-1}$: 3400, 2940, 2710, 1680.

Mass spectrum, m/z (%): 498 ($M^+$, 34), 359 (43), 262 (100).

The hydrochloride of the title compound, melting at 81 - 83°C, was prepared in a similar manner to that described in Example 2.

EXAMPLE 46

4(R)-[3-(4-Diphenylmethylene-1-piperidyl)propylcarbamoyl]-2-(4-pyridyl)thiazolidine and its hydrochloride

The title compound was prepared in a yield of 15% in a similar manner to that described in Example 17 by reacting 2-(4-pyridyl)thiazolidine-4(R)-carboxylic acid and 1-(3-aminopropyl)-4-(diphenylmethylene)piperidine (prepared as described in Preparation 15).

Infrared Absorption Spectrum ($CHC\ell_3$), $\nu_{max}$ $cm^{-1}$: 3050, 2935, 2567, 1665.

Mass spectrum, m/z (%): 496 ($M^+$-2, 50), 359 (39), 262 (100).

The hydrochloride of the title compound, melting at 84 - 85°C, was prepared in a similar manner to that described in Example 2.

EXAMPLE 47

4(R)-[3-(4-Diphenylmethyl-1-piperidyl)propylcarbamoyl}-2-(3-pyridyl)thiazolidine

The title compound, melting at 166 - 168°C, was prepared in a yield of 50% in a similar manner to that described in Example 17 by reacting 2-(3-pyridyl)thiazolidine-4(R)-carboxylic acid and 1-(3-aminopropyl)4-(diphenylmethyl)piperidine (prepared as described in Preparation 45).

Infrared Absorption Spectrum ($CHC\ell_3$), $\nu_{max}$ $cm^{-1}$: 3300, 2960, 1670, 1525.

Mass spectrum, m/z (%): 500 ($M^+$, 1), 264 (100).

PREPARATION 1

4-(Diphenylmethyl)-1-(3-phthalimidopropyl)piperazine

A mixture of 500 mg (1.98 mmole) of 4-(diphenylmethyl)piperazine, 530 mg (1.98 mmole) of N-(3-bromopropyl)phthalimide, 840 mg (7.92 mmole) of sodium carbonate, 10 mg of sodium iodide and 12 ml of methyl isobutyl ketone was heated under reflux overnight. The reaction mixture was then filtered, and the solvent was removed from the filtrate by evaporation under reduced pressure. The resulting residue was subjected to column chromatography through silica gel, using a 1 : 1 by volume mixture of ethyl acetate and hexane as the eluent, to afford 830 mg (yield 96%) of the title compound as an oil.

Infrared Absorption Spectrum ($CHC\ell_3$), $\nu_{max}$ $cm^{-1}$: 1710, 1395.

PREPARATIONS 2 TO 14

Using the corresponding cyclic amines, reactions were carried out in the same manner as described in Preparation 1 to obtain the following compounds.

PREPARATION 2

4-(Diphenylmethyl)-1-(4-phthalimidobutyl)piperazine

The title compound, melting at 125 - 129°C, was obtained in a yield of 90%.

Infrared Absorption Spectrum ($CHC\ell_3$), $\nu_{max}$ $cm^{-1}$: 1770, 1710, 1495, 1365.

PREPARATION 3

4-[Bis(4-fluorophenyl)methyl]-1-(2-phthalimidoethyl)piperazine

The title compound, melting at 125 - 126°C, was obtained in a yield of 77%.
Infrared Absorption Spectrum ($CHC\ell_3$), $\nu_{max}$ $cm^{-1}$: 1766, 1710, 1507, 1396.

PREPARATION 4

4-[Bis(4-fluorophenyl)methyl]-1-(3-phthalimidopropyl)piperazine

The title compound was obtained in a yield of 96%.
Infrared Absorption Spectrum (KBr), $\nu_{max}$ $cm^{-1}$: 1780, 1710, 1505, 1367.

PREPARATION 5

4-[Bis(4-fluorophenyl)methyl]-1-(4-phthalimidobutyl)piperazine

The title compound was obtained in a yield of 85%.
Infrared Absorption Spectrum ($CHC\ell_3$), $\nu_{max}$ $cm^{-1}$: 1770, 1710, 1505, 1395.

PREPARATION 6

4-[Bis(4-fluorophenyl)methyl]-1-(5-phthalimidopentyl)piperazine

The title compound was obtained in a yield of 70%.
Infrared Absorption Spectrum ($CHC\ell_3$), $\nu_{max}$ $cm^{-1}$: 1770, 1710, 1505, 1395.

PREPARATION 7

4-[α-(4-Chlorophenyl)benzyl]-1-(2-phthalimidoethyl)piperazine

The title compound was obtained in a yield of 89%.
Infrared Absorption Spectrum (KBr), $\nu_{max}$ $cm^{-1}$: 1770, 1710, 1396.

PREPARATION 8

4-[α-(4-Chlorophenyl)benzyl]-1-(3-phthalimidopropyl)piperidine

The title compound was obtained in a yield of 90%.
Infrared Absorption Spectrum ($CHC\ell_3$), $\nu_{max}$ $cm^{-1}$: 1770, 1710, 1395.

PREPARATION 9

4-(Diphenylmethylene)-1-(2-phthalimidoethyl)piperidine

The title compound, melting at 106 - 108°C, was obtained in a yield of 79%.
Infrared Absorption Spectrum (KBr), $\nu_{max}$ $cm^{-1}$: 1768, 1710, 1397.

PREPARATION 10

4-(Diphenylmethylene)-1-(3-phthalimidopropyl)piperidine

The title compound, melting at 108 - 110°C, was obtained in a yield of 91%.
Infrared Absorption Spectrum (KBr), $\nu_{max}$ $cm^{-1}$: 1770, 1705, 1403.

PREPARATION 11

4-(Diphenylmethylene)-1-(4-phthalimidobutyl)piperidine

The title compound, melting at 102 - 103°C, was obtained in a yield of 91%.
Infrared Absorption Spectrum (KBr), $\nu_{max}$ $cm^{-1}$: 1770, 1704, 1393.

PREPARATION 12

4-[Bis(4-fluorophenyl)methoxy]-1-(2-phthalimidoethyl)piperidine

The title compound was obtained in a yield of 70%.
Infrared Absorption Spectrum ($CHC\ell_3$), $\nu_{max}$ $cm^{-1}$: 1780, 1715, 1610.

PREPARATION 13

4-[Bis(4-fluorophenyl)methoxy]-1-(3-phthalimidopropyl)piperidine

The title compound was obtained in a yield of 95%.
Infrared Absorption Spectrum ($CHC\ell_3$), $\nu_{max}$ $cm^{-1}$: 1770, 1710, 1510, 1395.

PREPARATION 14

4-(α-Hydroxy-diphenylmethyl)-1-(3-phthalimidopropyl)piperidine

The title compound was obtained in a yield of 83%.
Infrared Absorption Spectrum (KBr), $\nu_{max}$ $cm^{-1}$: 1770, 1709, 1396.

PREPARATION 15

1-(3-Aminopropyl)-4-(diphenylmethylene)piperidine

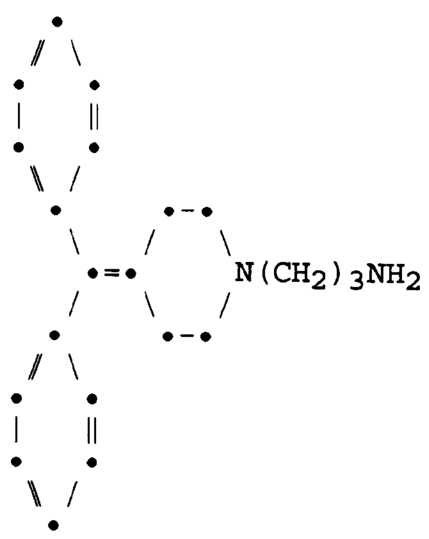

A mixture of 900 mg (2.06 mmole) of 4-(diphenylmethylene)-1-(3-phthalimidopropyl)piperidine (prepared as described in Preparation 10), 350 mg (7 mmole) of hydrazine hydrate and 20 ml of ethanol was heated under reflux for 2 hours. At the end of this time, the crystals which precipitated were filtered off and the solvent was removed from the filtrate by distillation under reduced pressure, to afford 460 mg (yield 73%) of the title compound as an oil.
Infrared Absorption Spectrum ($CHC\ell_3$), $\nu_{max}$ $cm^{-1}$: 2950, 2800, 1595,1490.

PREPARATIONS 16 TO 28

Using the corresponding phthalimide derivatives and hydrazine hydrate, reactions were carried out in the same manner as described in Preparation 15 to obtain the following compounds.

PREPARATION 16

1-(3-Aminopropyl)-4-(diphenylmethyl)piperazine

The title compound, melting at 62 - 63°C, was obtained in a yield of 22%.
Infrared Absorption Spectrum (KBr), $\nu_{max}$ $cm^{-1}$: 3024, 2949, 2803, 1596, 1450.

PREPARATION 17

1(4-Aminobutyl)-4-(diphenylmethyl)piperazine

The title compound was obtained in a quantitative yield.
Infrared Absorption Spectrum ($CHC\ell_3$), $\nu_{max}$ $cm^{-1}$: 3400, 2950, 2830, 1670, 1600, 1500.

PREPARATION 18

1-(2-Aminoethyl)-4-[bis(4-fluorophenyl)methyl]piperazine

The title compound was obtained in a yield of 45%.
Infrared Absorption Spectrum ($CHC\ell_3$), $\nu_{max}$ $cm^{-1}$: 3350, 2820, 1605, 1510.

PREPARATION 19

1(3-Aminopropyl)-4-[bis(4-fluorophenyl)methyl]piperazine

The title compound was obtained in a yield of 71%.
Infrared Absorption Spectrum ($CHC\ell_3$), $\nu_{max}$ $cm^{-1}$: 3350, 2820, 1605, 1510.

PREPARATION 20

1-(4-Aminobutyl)-4-[bis(4-fluorophenyl)methyl]piperazine

The title compound was obtained in a quantitative yield.
Infrared Absorption Spectrum ($CHC\ell_3$), $\nu_{max}$ $cm^{-1}$: 3200 (broad), 2930, 2820, 1605, 1505.

PREPARATION 21

1-(5-Aminopentyl)-4-[bis(4-fluorophenyl)methyl]piperazine

The title compound was obtained in a yield of 93%.
Infrared Absorption Spectrum ($CHC\ell_3$), $\nu_{max}$ $cm^{-1}$: 3500 - 3100 (broad), 2940, 2830, 1640, 1605, 1505.

PREPARATION 22

1-(2-Aminoethyl)-4-[$\alpha$-(4-chlorophenyl)benzyl]piperazine

The title compound was obtained in a yield of 89%.
Infrared Absorption Spectrum ($CHC\ell_3$), $\nu_{max}$ $cm^{-1}$: 3200 (broad), 2950, 2830, 1490.

PREPARATION 23

1-(3-Aminopropyl)-4-[α-(4-chlorophenyl)benzyl]piperazine

The title compound was obtained in a yield of 68%.
Infrared Absorption Spectrum ($CHCℓ_3$), $\nu_{max}$ $cm^{-1}$: 3200, 2950, 2830, 1490.

PREPARATION 24

1-(2-Aminoethyl)-4-(diphenylmethylene)piperidine

The title compound was obtained in a yield of 42%.
Infrared Absorption Spectrum ($CHCℓ_3$), $\nu_{max}$ $cm^{-1}$:3600 - 3160 (broad), 2950, 2820, 1650, 1595, 1495.

PREPARATION 25

1-(4-Aminobutyl)-4-(diphenylmethylene)piperidine

The title compound was obtained in a quantitative yield.
Infrared Absorption Spectrum ($CHCℓ_3$), $\nu_{max}$ $cm^{-1}$: 3500 - 3100 (broad), 2940, 1640, 1600, 1570, 1495.

PREPARATION 26

1-(2-Aminoethyl)-4-[bis(4-fluorophenyl)methoxyl]piperidine

The title compound was obtained in a quantitative yield.
Infrared Absorption Spectrum ($CHCℓ_3$), $\nu_{max}$ $cm^{-1}$: 3400, 2970, 1610, 1515.

PREPARATION 27

1-(3-Aminopropyl)-4-[bis(4-fluorophenyl)methoxy]piperidine

The title compound was obtained in a yield of 85%.
Infrared Absorption Spectrum ($CHCℓ_3$), $\nu_{max}$ $cm^{-1}$: 3200, 2950, 1610, 1510.

PREPARATION 28

1-(3-Aminopropyl)-4-(α-hydroxydiphenylmethyl)piperidine

The title compound, melting at 102 - 104°C, was obtained in a yield of 93%.
Infrared Absorption Spectrum (KBr), $\nu_{max}$ $cm^{-1}$: 3348, 3257, 2938, 2816, 1489, 1447.

PREPARATION 29

2-(3-Pyridyl)thiazolidine-4(S)-carboxylic acid

A solution of 1.07 g of pyridine-3-aldehyde and 1.21 g of D-cysteine in 60% v/v aqueous ethanol was heated under reflux for 4 hours. The mixture was cooled to room temperature and the resulting solid material was removed by filtration. The filtrate was concentrated by evaporation under reduced pressure, and 5 ml of ethanol were added to the resulting residue. Collecting the precipitated crystals by filtration gave 1.13 g (yield 54%) of the title compound, melting at 138 - 139°C.
Infrared Absorption Spectrum (KBr), $\nu_{max}$ $cm^{-1}$: 3260, 2920, 2400, 1720, 1200.

PREPARATION 30

4-Diphenylmethylene-1-[2-(N-methylamino)ethyl]piperidine

A solution of 1.13 g of 4-diphenylmethylene-1-(2-N-formamidoethyl)piperidine (prepared as described in

Preparation 31) in 7 ml of tetrahydrofuran was added dropwise to a suspension of 140 mg of lithium aluminium hydride in 10 ml of tetrahydrofuran at room temperature under a nitrogen atmosphere, and the mixture was heated under reflux for 1 hour. At the end of this time, the mixture was cooled to room temperature and sodium sulphate decahydrate was added to the mixture to decompose the excess lithium aluminium hydride. The insoluble material was removed by filtration. Ethyl acetate was added to the filtrate, and the mixture was washed with water and with a saturated aqueous solution of sodium chloride, in that order, after which it was dried over anhydrous magnesium sulphate. Evaporation of solvent under reduced pressure gave 830 mg (yield 80%) of the title compound as an oil.

Infrared Absorption Spectrum ($CHC\ell_3$), $\nu_{max}$ $cm^{-1}$: 2875, 1500, 1440, 1100.

PREPARATION 31

4-Diphenylmethylene-1-(2-N-formamidoethyl)piperidine

A solution of 1.0 g of 1-(2-aminoethyl)-4-(diphenylmethylene)piperidine (prepared as described in Preparation 24) in 10 ml of ethyl formate was heated under reflux overnight. At the end of this time, the mixture was cooled to room temperature and the solvent was removed by evaporation under reduced pressure. The residue was purified by column chromatography through silica gel, using a 40 : 1 by volume mixture of chloroform and methanol as the eluent, to give 1.13 g (a quantitative yield) of the title compound as an oil.

Infrared Absorption Spectrum ($CHC\ell_3$), $\nu_{max}$ $cm^{-1}$: 3400, 3000, 1680, 1490.

PREPARATION 32

4-Diphenylmethylene-1-[3-(N-ethylamino)propyl]piperidine

A solution of 1.14 g of 1-(3-acetamidopropyl)-4-(diphenylmethylene)piperidine (prepared as described in Preparation 33) in 7 ml of tetrahydrofuran was added dropwise to a suspension of 140 mg of lithium aluminium hydride in 10 ml of tetrahydrofuran at room temperature under a nitrogen atmosphere, and the mixture was heated under reflux for 4 hours. At the end of this time, the mixture was cooled to room temperature, and sodium sulphate decahydrate was added to the mixture to decompose excess lithium aluminium hydride. The insoluble material was removed by filtration. Ethyl acetate was added to the filtrate, and the mixture was washed with water and with a saturated aqueous solution of sodium chloride, in that order, after which it was dried over anhydrous magnesium sulphate. Evaporation of solvent under reduced pressure gave 710 mg (yield 65%) of the title compound as an oil.

Infrared Absorption Spectrum ($CHC\ell_3$), $\nu_{max}$ $cm^{-1}$: 2950, 2850, 1500, 1440, 1120.

PREPARATION 33

1-(3-Acetamidopropyl)-4-(diphenylmethylene)piperidine

680 mg of triethylamine was added to a solution ofl1.0 g of 1-(3-aminopropyl)-4-(diphenylmethylene)piperidine (prepared as described in Preparation 15) in 10 ml of methylene chloride at room temperature; a solution of 680 mg of acetyl chloride in 2 ml of methylene chloride was then added dropwise to it at -10°C, after which the mixture was stirred at the same temperature for 5 minutes. Methylene chloride was then added to the mixture, which was then washed with water and with a saturated aqueous solution of sodium chloride, in that order, after which it was dried over anhydrous sodium sulphate. Evaporation of the solvent under reduced pressure gave 1.14 g (a quantitative yield) of the title compound as an oil.

Infrared Absorption Spectrum (liquid film), $\nu_{max}$ $cm^{-1}$: 3350, 2815, 1650, 1537.

PREPARATION 34

2-(N-Ethoxycarbonyl-N-methylamino)ethyl methanesulphonate

1.65 g of methanesulphonyl chloride was added dropwise, with stirring, to a solution of 1.66 g of 2-(N-ethoxycarbonyl-N-methylamino)ethanol and 1.65 g of triethylamine in 20 ml of methylene chloride, whilst ice-cooling, and the solution was then stirred at the same temperature for 30 minutes. At the end of this time, the mixture was poured into ice-water and extracted with methylene chloride. The extracts were combined and washed with water, after which they were dried over anhydrous sodium sulphate. Evaporation of the solvent

under reduced pressure gave a residue which was purified by column chromatography through silica gel, using a 2 : 1 by volume mixture of ethyl acetate and hexane as the eluent, to give 2.62 g (yield 81%) of the title compound as an oil.

Infrared Absorption Spectrum ($CHC\ell_3$), $\nu_{max}$ $cm^{-1}$: 3000, 1750, 1590, 1485.

PREPARATION 35

4-Diphenylmethyl-1-[2-(N-ethoxycarbonyl-N-methylamino)ethyl]piperazine

A mixture of 0.23 g of 2-(N-ethoxycarbonyl-N-methylamino)ethyl methanesulphonate (prepared as described in Preparation 34) and 0.26 g of 4-diphenylmethylpiperazine was stirred at 80°C for 5 hours. At the end of this time, the mixture was purified by column chromatography through silica gel, using a 20 : 1 by volume mixture of ethyl acetate and ethanol as the eluent, to give 0.25 g (yield 64%) of the title compound as an oil.

Infrared Absorption Spectrum ($CHC\ell_3$), $\nu_{max}$ $cm^{-1}$:3010, 2930, 2820, 1750, 1690, 1490.

PREPARATION 36

4-Diphenylmethyl-1-[2-(N-methylamino)ethyl]piperazine

A solution of 1.6 g of 4-diphenylmethyl-1-[2-(N-ethoxycarbonyl-N-methylamino)ethyl]piperazine (prepared as described in Preparation 35) in 20 ml of a 10% w/v aqueous solution of potassium hydroxide and 20 ml of ethylene glycol was stirred at 140°C for 20 hours. At the end of this time, the mixture was cooled to room temperature, poured into ice-water and extracted with methylene chloride. The extracts were combined, washed with water and dried over anhydrous sodium sulphate. Evaporation of the solvent under reduced pressure gave 1.14 g (yield 88%) of the title compound as an oil.

Infrared Absorption Spectrum ($CHC\ell_3$), $\nu_{max}$ $cm^{-1}$: 2970, 2830, 1610, 1495.

PREPARATION 37

2-[5-(2-Methylpyridyl)]thiazolidine-4(R)-carboxylic acid

The title compound, melting at 147 - 148°C, was prepared in a yield of 81% in a similar manner to that described in Preparation 29 by reacting 2-methyl-5-pyridinealdehyde and L-cysteine.

Infrared Absorption Spectrum (KBr), $\nu_{max}$ $cm^{-1}$: 3260, 2920, 2420, 1950, 1720, 1610.

PREPARATION 38

4-[α-(2-Pyridyl)benzyl]-1-(2-cyanoethyl)piperazine

1.05 g of N,N-diisopropyl-N-ethylamine was added, with stirring, to a solution of 2.0 g of 4-[α-(2-pyridyl)benzyl]piperazine and 1.0 g of 3-bromopropionitrile in 20 ml of methylene chloride, whilst ice-cooling, and the mixture was stirred at room temperature for 16 hours. At the end of this time, the mixture was poured into ice-water and extracted with chloroform. The extracts were combined and dried over anhydrous sodium sulphate, after which the solvent was removed by evaporation under reduced pressure. The resulting residue was purified by column chromatography through silica gel, using a 20 : 1 by volume mixture of ethyl acetate and ethanol as the eluent, to give 2.1 g (yield 88%) of the title compound as crystals, melting at 114 - 116°C.

Infrared Absorption Spectrum ($CHC\ell_3$), $\nu_{max}$ $cm^{-1}$: 2950, 2825, 2250, 1590, 1575, 1490, 1465, 1455, 1439.

PREPARATION 39

4-(Diphenylmethylene)-1-(4-cyanobutyl)piperidine

The title compound was prepared in a yield of 50% in a similar manner to that described in Preparation 38 by reacting 4-(diphenylmethylene)piperidine and 4-bromobutylnitrile.

Infrared Absorption Spectrum ($CHC\ell_3$), $\nu_{max}$ $cm^{-1}$: 2950, 2850, 1500, 1450.

PREPARATION 40

4-(Diphenylmethylene)-1-(6-cyanohexyl)piperidine

The title compound was prepared in a yield of 53% in a similar manner to that described in Preparation 38 by reacting 4-(diphenylmethylene)piperidine and 6-bromohexylnitrile.

Infrared Absorption Spectrum ($CHC\ell_3$), $\nu_{max}$ $cm^{-1}$: 2930, 2860, 2520, 2240, 1600, 1490.

PREPARATION 41

4-[α-(2-Pyridyl)benzyl]-1-(3-aminopropyl)piperazine

A solution of 2.0 g of 4-[α-(2-pyridyl)benzyl]-1-(2-cyanoethyl)piperazine (prepared as described in Preparation 38) in 30 ml of tetrahydrofuran was added dropwise to a suspension of 0.24 g of lithium aluminium hydride in 30 ml of tetrahydrofuran at 5 - 7°C, and the mixture was stirred at room temperature for 30 minutes. At the end of this time, excess lithium aluminium hydride was decomposed by adding a 4% w/v aqueous solution of sodium hydroxide, and the insoluble material was removed by filtration. The filtrate was dried over anhydrous sodium sulphate, and then the solvent was removed by evaporation under reduced pressure, to give 1.8 g (yield 90%) of the title compound as an oil.

Infrared Absorption Spectrum ($CHC\ell_3$), $\nu_{max}$ $cm^{-1}$: 3250, 2950, 2825, 1660, 1590.

PREPARATION 42

1-(5-Aminopentyl)-4-(diphenylmethylene)piperidine

The title compound was prepared in a yield of 36% in a similar manner to that described in Preparation 41 by reacting 4-(diphenylmethylene)-1-(4-cyanobutyl)piperidine (prepared as described in Preparation 39) and lithium aluminium hydride.

Infrared Absorption Spectrum ($CHC\ell_3$), $\nu_{max}$ $cm^{-1}$: 3400, 2950, 1670, 1530, 1450.

PREPARATION 43

1-(7-Aminoheptyl)-4-(diphenylmethylene)piperidine

The title compound was prepared in a yield of 91% in a similar manner to that described in Preparation 41 by reacting 4-(diphenylmethylene)-1-(6-cyanohexyl)piperidine (prepared as described in Preparation 40) and lithium aluminium hydride.

Infrared Absorption Spectrum ($CHC\ell_3$), $\nu_{max}$ $cm^{-1}$: 3060, 2930, 2850, 2800, 1665, 1595.

PREPARATION 44

4-Diphenylmethyl-1-(3-phthalimidopronyl)piperidine

A mixture of 1.8 g of 4-diphenylmethylpiperidine, 1.92 g of N-(3-bromopropyl)phthalimide, 3.0 g of sodium carbonate and 20 mg of sodium iodide in 70 ml of methyl isobutyl ketone was heated under reflux for 5 hours. At the end of this time, the mixture was cooled to room temperature and filtered. The filtrate was concentrated by evaporation under reduced pressure, and the resulting residue was purified by column chromatography through silica gel, using a 1 : 1 by volume mixture of hexane and ethyl acetate as the eluent, to give 3.0 g (yield 95%) of the title compound as crystals, melting at 104 - 106°C.

Infrared Absorption Spectrum ($CHC\ell_3$), $\nu_{max}$ $cm^{-1}$: 2960, 1775, 1715, 1445, 1400.

PREPARATION 45

1-(3-Aminopropyl)-4-diphenylmethylpiperidine

A mixture of 2.8 g of 4-diphenylmethyl-1-(3-phthalimidopropyl)piperidine (prepared as described in Preparation 44) and 0.90 g of hydrazine hydrate in 100 ml of ethanol was heated under reflux for 2 hours. At the end of this time, the mixture was cooled to room temperature and filtered. The filtrate was concentrated by

evaporation under reduced pressure, to give 0.93 g (yield 47%) of the title compound as an oil.

Infrared Absorption Spectrum ($CHCl_3$), $\nu_{max}$ $cm^{-1}$ 3400, 2970, 1670, 1600, 1495, 1455.

PREPARATION 46

4-[Bis(4-fluorophenyl)-α-hydroxymethyl]-1-(2-phthalimidoethyl)piperidine

The title compound was prepared in a yield of 78% in a similar manner to that described in Preparation 1 by reacting N-(2-bromoethyl)phthalimide and 4-[bis(4-fluorophenyl)-α-hydroxymethyl]piperidine

Infrared Absorption Spectrum ($CHCl_3$), $\nu_{max}$ $cm^{-1}$: 2940, 2800, 1770, 1710, 1600, 1505.

PREPARATION 47

4-Diphenylmethylene-1-(2-phthalimidoethyl)piperidine

The title compound was prepared in a yield of 88% in a similar manner to that described in Preparation 1 by reacting N-(2-bromoethyl)phthalimide and 4-diphenylmethylenepiperidine.

Infrared Absorption Spectrum ($CHCl_3$), $\nu_{max}$ $cm^{-1}$: 2950, 2800, 1770, 1710, 1600, 1505.

PREPARATION 48

4-[Bis(4-fluorophenyl)methylene]-1-(3-phthalimidopropyl)piperidine

The title compound was prepared in a yield of 97% in a similar manner to that described in Preparation 1 by reacting N-(3-bromopropyl)phthalimide and 4-[bis(4-fluorophenyl)methylene]piperidine.

Infrared Absorption Spectrum ($CHCl_3$), $\nu_{max}$ $cm^{-1}$: 2950, 2800, 1775, 1715, 1605, 1505.

PREPARATION 49

4-[α-(3-Pyridyl)benzyl]-1-(2-phthalimidoethyl)piperazine

The title compound was prepared in a yield of 53% in a similar manner to that described in Preparation 1 by reacting N-(2-bromoethyl)phthalimide and 4-[α-(3-pyridyl)benzyl]piperazine.

Infrared Absorption Spectrum ($CHCl_3$), $\nu_{max}$ $cm^{-1}$:2950, 2810, 1775, 1710, 1395.

PREPARATION 50

4-[α-(4-Pyridyl)benzyl]-1-(2-phthalimidoethyl)piperazine

The title compound was prepared in a yield of 77% in a similar manner to that described in Preparation 1 by reacting N-(2-bromoethyl)phthalimide and 4-[α-(4-pyridyl)benzyl]piperazine.

Infrared Absorption Spectrum ($CHCl_3$), $\nu_{max}$ $cm^{-1}$: 2950, 2820, 1775, 1710, 1595, 1395.

PREPARATION 51

4-[α-(2-Pyridyl)-4-fluorobenzyl]-1-(2-phthalimidoethyl)piperazine

The title compound was prepared in a yield of 80% in a similar manner to that described in Preparation 1 by reacting N-(2-bromoethyl)phthalimide and 4-[α-(2-pyridyl)-4-fluorobenzyl]piperazine.

Infrared Absorption Spectrum ($CHCl_3$), $\nu_{max}$ $cm^{-1}$: 2900, 1625, 1595, 1575.

PREPARATION 52

4-[α-(2-Pyridyl)-4-trifluoromethylbenzyl]-1-(2-phthalimidoethyl)piperazine

The title compound was prepared in a yield of 87% in a similar manner to that described in Preparation 1 by reacting N-(2-bromoethyl)phthalimide and 4-[α-(2-pyridyl)-4-trifluoromethylbenzyl]piperazine.

Infrared Absorption Spectrum ($CHCl_3$), $\nu_{max}$ $cm^{-1}$: 2960, 2830, 1775, 1715, 1595, 1330.

PREPARATION 53

4-[α-(2-Pyridyl)benzyl]-1-(2-phthalimidoethyl)piperazine

The title compound, melting at 114 - 117°C, was prepared in a yield of 90% in a similar manner to that described in Preparation 1 by reacting N-(2-bromoethyl) phthalimide and 4-[α-(2-pyridyl)benzyl]piperazine.

Infrared Absorption Spectrum (CHCℓ$_3$), $\nu_{max}$ cm$^{-1}$: 2950, 2800, 1780, 1710, 1590, 1400.

PREPARATION 54

4-[α-(2-Thienyl)benzylidene]-1-(3-phthalimidopropyl)piperidine

The title compound was prepared in a yield of 84% in a similar manner to that described in Preparation 1 by reacting N-(3-bromopropyl)phthalimide and 4-[α-(2-thienyl)benzylidene]piperidine.

Infrared Absorption Spectrum (CHCℓ$_3$), $\nu_{max}$ cm$^{-1}$: 3000, 2940, 2900, 2805, 1595, 1490, 1440.

PREPARATION 55

1-(2-Aminoethyl)-4-[bis(4-fluorophenyl)-α-hydroxymethyl]piperidine

The title compound was prepared in a yield of 98% in a similar manner to that described in Preparation 15 by reacting 4-[bis(4-fluorophenyl)-α-hydroxymethyl]-1-(2-phthalimidoethyl)piperidine (prepared as described in Preparation 46) and hydrazine hydrate.

Infrared Absorption Spectrum (CHCℓ$_3$), $\nu_{max}$ cm$^{-1}$: 2950, 2810, 1605, 1505.

PREPARATION 56

1-(2-Aminoethyl)-4-[bis(4-fluorophenyl)methylene]piperidine

The title compound was prepared in a yield of 97% in a similar manner to that described in Preparation 15 by reacting 4-[bis(4-fluorophenyl)methylene]-1-(2-phthalimidoethyl)piperidine (prepared as described in Preparation ??) and hydrazine hydrate.

Infrared Absorption Spectrum (CHCℓ$_3$), $\nu_{max}$ cm$^{-1}$: 2950, 2810, 1605, 1505.

PREPARATION 57

1-(3-Aminopropyl)-4-[bis(4-fluorophenyl)methylene]piperidine

The title compound was prepared in a yield of 20% in a similar manner to that described in Preparation 15 by reacting 4-[bis(4-fluorophenyl)methylene]-1-(3-phthalimidopropyl)piperidine (prepared as described in Preparation 48) and hydrazine hydrate.

Infrared Absorption Spectrum (CHCℓ$_3$), $\nu_{max}$ cm$^{-1}$: 3370, 3270, 3170, 2940, 2820, 1660, 1605, 1505.

PREPARATION 58

1-(2-Aminoethyl)-4-[α-(3-pyridyl)benzyl]piperazine

The title compound was prepared in a yield of 98% in a similar manner to that described in Preparation 15 by reacting 4-[α-(3-pyridyl)benzyl]-1-(2-phthalimidoethyl)piperazine (prepared as described in Preparation 49) and hydrazine hydrate.

Infrared Absorption Spectrum (CHCℓ$_3$), $\nu_{max}$ cm$^{-1}$: 3380, 3300, 3200, 2970, 2830, 1670, 1605, 1590, 1580.

PREPARATION 59

1-(2-Aminoethyl)-4-[α-(4-pyridyl)benzyl]piperazine

The title compound was prepared in a quantitative yield in a similar manner to that described in Preparation 15 by reacting 4-[α-(4-pyridyl)benzyl]-1-(2-phthalimidoethyl)piperazine (prepared as described in Preparation

50) and hydrazine hydrate.

Infrared Absorption Spectrum ($CHC\ell_3$), $\nu_{max}$ $cm^{-1}$: 3380, 3300, 3200, 2970, 2830, 1670, 1600.

PREPARATION 60

1-(2-Aminoethyl)-4-[α-(2-pyridyl)-4-fluorobenzyl]piperazine

The title compound was prepared in a yield of 88% in a similar manner to that described in Preparation 15 by reacting 4-[α-(2-pyridyl)-4-fluorobenzyl]-1-(2-phthalimidoethyl)piperazine (prepared as described in Preparation 51) and hydrazine hydrate.

Infrared Absorption Spectrum ($CHC\ell_3$), $\nu_{max}$ $cm^{-1}$: 3380, 3300, 3200, 2970, 2830, 1610.

PREPARATION 61

1-(2-Aminoethyl)-4-[α-(2-pyridyl)-4-trifluoromethylbenzyl]piperazine

The title compound was prepared in a quantitative yield in a similar manner to that described in Preparation 15 by reacting 4-[α-(2-pyridyl)-4-trifluoromethylbenzyl]-1-(2-phthalimidoethyl)piperazine (prepared as described in Preparation 52) and hydrazine hydrate.

Infrared Absorption Spectrum ($CHC\ell_3$), $\nu_{max}$ $cm^{-1}$: 3380, 3300, 3200, 2960, 2830, 1620.

PREPARATION 62

1-(2-Aminoethyl)-4-[α-(2-pyridyl)benzyl]piperazine

The title compound was prepared in a yield of 52% in a similar manner to that described in Preparation 15 by reacting 4-[α-(2-pyridyl)benzyl]-1-(2-phthalimidoethyl)piperazine (prepared as described in Preparation 53) and hydrazine hydrate.

Infrared Absorption Spectrum ($CHC\ell_3$), $\nu_{max}$ $cm^{-1}$: 3400, 3000, 2850, 1640.

PREPARATION 63

1-(3-Aminopropyl)-4-[α-(2-thienyl)benzylidene]piperidine

The title compound was prepared in a yield of 92% in a similar manner to that described in Preparation 15 by reacting 4-[α-(2-thienyl)benzylidene]-1-(3-phthalimidopropyl)piperidine (prepared as described in Preparation 54) and hydrazine hydrate.

Infrared Absorption Spectrum ($CHC\ell_3$), $\nu_{max}$ $cm^{-1}$:3200, 3030, 2950, 2800, 1660, 1595.

## Claims

1. Compounds of formula (I):

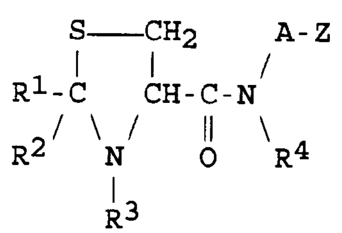

(I)

in which:

$R^1$ represents a pyridyl group which is unsubstituted or which is substituted by at least one alkyl group having from 1 to 4 carbon atoms and/or alkoxy group having from 1 to 4 carbon atoms;

$R^2$ represents a hydrogen atom, an alkyl group having from 1 to 4 carbon atoms, or a pyridyl group which is unsubstituted or which is substituted by at least one alkyl group having from 1 to 4 carbon atoms and/or

alkoxy group having from 1 to 4 carbon atoms;
$R^3$ represents a hydrogen atom, an alkyl group having from 1 to 4 carbon atoms, an alkoxycarbonyl group having from 2 to 5 carbon atoms, an aralkyloxycarbonyl group in which the alkyl part has from 1 to 4 carbon atoms and the aryl part is as defined below, an aryloxycarbonyl group in which the aryl part is as defined below, an aliphatic carboxylic acyl group having from 1 to 5 carbon atoms, an aliphatic carboxylic acyl group which has from 2 to 5 carbon atoms and which is substituted by at least one halogen atom, an arylcarbonyl group in which the aryl part is as defined below, an alkylsulphonyl group having from 1 to 4 carbon atoms, an arylsulphonyl group in which the aryl part is as defined below, or a group of formula $-B-NR^5R^6$, in which

B represents an alkylene or alkylidene group having from 2 to 4 carbon atoms; and

$R^5$ and $R^6$ are the same or different and each represents a hydrogen atom or an alkyl group having from 1 to 4 carbon atoms;

said aryl groups have from 6 to 10 ring atoms and are unsubstituted or are substituted by at least one alkyl group having from 1 to 4 carbon atoms, and/or alkoxy group having from 1 to 4 carbon atoms and/or halogen atom;
$R^4$ represents a hydrogen atom or an alkyl group having from 1 to 4 carbon atoms;
A represents an alkylene or alkylidene group having from 2 to 7 carbon atoms; and
Z represents a group of formula:

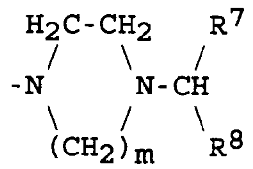

(II)

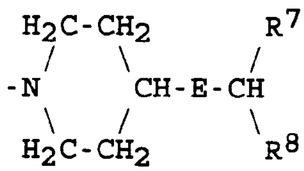

(III)

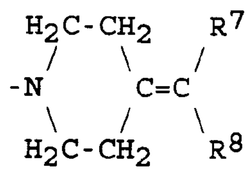

(IV)

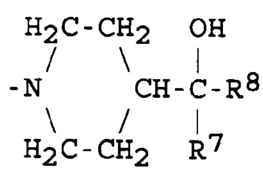

(V)

or a group of formulae (II), (III), (IV) or (V) in which one or more of the ring atoms is substituted by an alkyl group having from 1 to 4 carbon atoms;
in which:

$R^7$ and $R^8$ are the same or different and each represents an unsubstituted phenyl group, a substituted phenyl group which is substituted by at least one of substituents (a), defined below, or an aromatic heterocyclic group which has 5 or 6 ring atoms of which 1 or 2 are nitrogen and/or oxygen and/or sulphur hetero-atoms, said aromatic heterocyclic group being unsubstituted or being substituted by at least one of substituents (a), defined below;

said substituents (a) are selected from alkyl groups having from 1 to 4 carbon atoms, alkoxy groups having from 1 to 4 carbon atoms, halogen atoms and haloalkyl groups having from 1 to 4 carbon atoms;

E represents a direct carbon-carbon single bond or an oxygen atom; and

m is 2 or 3;
and pharmaceutically acceptable salts thereof.

2. A compound according to Claim 1, in which $R^1$ represents an unsubstituted pyridyl group or a substituted pyridyl group having at least one alkyl substituent having from 1 to 4 carbon atoms.

3. A compound according to Claim 1 or Claim 2, in which $R^2$ represents a hydrogen atom.

4. A compound according to any one of Claims 1 to 3, in which $R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms.

5. A compound according to Claim 4, in which $R^3$ represents a hydrogen atom.

6. A compound according to any one of Claims 1 to 5, in which $R^4$ represents a hydrogen atom.

7. A compound according to any one of Claims 1 to 6, in which Z represents a 4-(diphenylmethyl)-1-piperazinyl, 4-[α-(chlorophenyl)benzyl]-1-piperazinyl, 4-[bis(fluorophenyl)methyl]-1-piperazinyl, 4-[α-(chlorophenyl)-o-, m- or p-fluorobenzyl]-1-piperazinyl, 4-[bis(chlorophenyl)methyl]-1-piperazinyl, 4-(diphenylmethyl)-1-piperidyl, 4-[bis(fluorophenyl)methyl]-1-piperidyl, 4-[α-(chlorophenyl)benzyl]-1-piperidyl, 4-(diphenylmethoxy)-1-piperidyl, 4-[α-(fluorophenyl)benzyloxy]-1-piperidyl, 4-[bis(fluorophenyl)methoxy]-1-piperidyl, 4-[α-(chlorophenyl)benzyloxy]-1-piperidyl, 4-(diphenylmethylene)-1-piperidyl, 4-[α-(fluorophenyl)benzylidene]-1-piperidyl, 4-[bis(fluorophenyl)methylene]-1-piperidyl, 4-[α-(chlorophenyl)benzylidene]-1-piperidyl, 4-(α-hydroxydiphenylmethyl)-1-piperidyl, 4-[α-(fluorophenyl)-α-hydroxybenzyl]-1-piperidyl, 4-[bis(fluorophenyl)-α-hydroxymethyl]-1-piperidyl or 4-[α-(chlorophenyl)-α-hydroxybenzyl]-1-piperidyl group.

8. A compound according to Claim 1, in which:
$R^1$ represents a pyridyl group which is unsubstituted or which is substituted by at least one alkyl substituent having from 1 to 4 carbon atoms;
$R^2$ represents a hydrogen atom;
$R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms;
$R^4$ represents a hydrogen atom or an alkyl group having from 1 to 4 carbon atoms;
A represents an alkylene or alkylidene group having from 2 to 7 carbon atoms; and
Z represents a group of formula (II), (III), (IV) or (V), as defined in Claim 1, in which:
$R^7$ and $R^8$ are the same or different and each represents an unsubstituted phenyl group, a substituted phenyl group which is substituted by at least one of substituents (a′), defined below, or an aromatic heterocyclic group which has 5 or 6 ring atoms of which 1 is a nitrogen and/or oxygen and/or sulphur hetero-atom, said aromatic heterocyclic group being unsubstituted or being substituted by at least one alkyl substituent having from 1 to 4 carbon atoms;
said substituents (a′) are alkyl groups having from 1 to 4 carbon atoms, halogen atoms or trifluoromethyl groups;
E represents an oxygen atom; and
m is 2.

9. A compound according to Claim 1, in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a group of formula (II), (III), (IV) or (V), as defined in Claim 1, in which:
$R^7$ and $R^8$ are the same or different and each represents an unsubstituted phenyl group, a substituted phenyl group which is substituted by at least one of substituents (a″), defined below, a pyridyl group or a thienyl group;
said substituents (a″) are methyl groups, fluorine atoms or chlorine atoms;
E represents an oxygen atom; and

m is 2.

**10.** A compound according to Claim 1, in which:
$R^1$ represents a pyridyl group which is unsubstituted or which is substituted by at least one alkyl substituent having from 1 to 4 carbon atoms;
$R^2$ represents a hydrogen atom;
$R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms;
$R^4$ represents a hydrogen atom or an alkyl group having from 1 to 4 carbon atoms;
A represents an alkylene or alkylidene group having from 2 to 6 carbon atoms; and
Z represents a group of formula (II), (III), (IV) or (V), in which:
$R^7$ and $R^8$ are the same or different and each represents an unsubstituted phenyl group or a substituted phenyl group which is substituted by at least one of substituents (a‴), defined below;
said substituents (a‴) are alkyl groups having from 1 to 4 carbon atoms or halogen atoms;
E represents a direct carbon-carbon single bond or an oxygen atom; and
m is 2 or 3.

**11.** A compound according to Claim 1, in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a group of formula (II), (III), (IV) or (V), in which:
$R^7$ and $R^8$ are the same or different and each represents an unsubstituted phenyl group or a substituted phenyl group which is substituted by at least one of substituents (a″), defined below;
said substituents (a″) are methyl groups, fluorine atoms or chlorine atoms;
E represents an oxygen atom; and
m is 2.

**12.** A compound according to Claim 1, in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a 4-[α-(chlorophenyl)benzyl]-1-piperazinyl, 4-[bis(fluorophenyl)methyl]-1-piperazinyl, 4-(diphenylmethylene)-1-piperidyl, 4-[bis(fluorophenyl)methoxy]-1-piperidyl or 4-(α-hydroxydiphenylmethyl)-1-piperidyl group.

**13.** The following compounds according to Claim 1:
{3-[4-bis(4-fluorophenyl)methyl-1-piperazinyl] propylcarbamoyl}-2-(3-pyridyl)thiazolidine;
{3-[4-(α-2-pyridylbenzyl)-1-piperazinyl]propylcarbamoyl}-2-(3-pyridyl)thiazolidine;
[2-(4-diphenylmethyl-1-piperazinyl)ethylcarbamoyl]-2-(3-pyridyl)thiazolidine;
[3-{4-[α-(4-chlorophenyl)benzyl]-1-piperazinyl}propylcarbamoyl]-2-(3-pyridyl)thiazolidine;
[2-{4-[α-(4-chlorophenyl)benzyl]-1-piperazinyl}ethylcarbamoyl]-2-(3-pyridyl)thiazolidine;
{3-[4-bis(4-fluorophenyl)methoxy-1-piperidyl]propylcarbamoyl}-2-(3-pyridyl)thiazolidine;
{2-[4-(diphenylmethylene)-1-piperidyl]ethylcarbamoyl}-2-(3-pyridyl)thiazolidine;
{3-[4-(diphenylmethylene)-1-piperidyl]propylcarbamoyl}-2-(3-pyridyl)thiazolidine;
{4-[4-(diphenylmethylene)-1-piperidyl]butylcarbamoyl}-2-(3-pyridyl)thiazolidine;
[3-{4-[bis(4-fluorophenyl)methylene]-1-piperidyl}propylcarbamoyl]-2-(3-pyridyl)thiazolidine;
{3-[4-(α-2-thienyl)benzylidene-1-piperidyl]propylcarbamoyl}-2-(3-pyridyl)thiazolidine;
[N-{3-[4-diphenylmethylene-1-piperidyl]propyl}-N-ethylcarbamoyl]-2-(3-pyridyl)thiazolidine;
[N-{2-[4-diphenylmethylene-1-piperidyl]ethyl}-Nmethylcarbamoyl]-2-(3-pyridyl)thiazolidine; and
{2-[4-(α-hydroxydiphenylmethyl)-1-piperidyl]ethylcarbamoyl}-2-(3-pyridyl)thiazolidine;
and salts thereof.

**14.** A composition for the treatment or prophylaxis of histamine- or PAF- related disorders, which comprises

an anti-histamine or anti-PAF agent in admixture with a pharmaceutically acceptable carrier or diluent, in which the anti-histamine or anti-PAF agent is at least one compound according to any one of Claims 1 to 13.

**15.** The use of a compound according to any one of Claims 1 to 13 in therapy.

**16.** The use of a compound according to any one of Claims 1 to 13 for the manufacture of a medicament for the treatment or prophylaxis of histamine-related disorders.

**17.** A process for preparing a compound according to any one of Claims 1 to 13, which comprises reacting a compound of formula (VI):

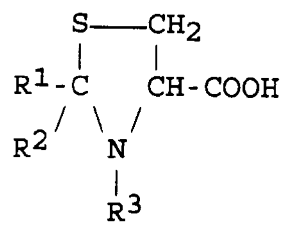

(VI)

(in which $R^1$, $R^2$ and $R^3$ are as defined in Claim 1) or a reactive derivative thereof with a compound of formula (VII):

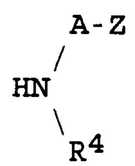

(VII)

(in which A, Z and $R^4$ are as defined in Claim 1) or a compound of formula (VI) or (VII) or reactive derivative thereof in which a reactive group in one or more of the substituents Z, $R^1$, $R^2$, $R^3$ and $R^4$ is protected,and, if desired, removing protecting groups from the resulting product, and, if desired, salifying the compound of formula (I).

**Claims for the following Contracting States: ES. GR.**

**1.** A process for preparing a compound of formula (I):

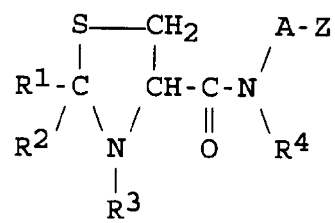

(I)

[in which:
$R^1$ represents a pyridyl group which is unsubstituted or which is substituted by at least one alkyl group having from 1 to 4 carbon atoms and/or alkoxy group having from 1 to 4 carbon atoms;
$R^2$ represents a hydrogen atom, an alkyl group having from 1 to 4 carbon atoms, or a pyridyl group which is unsubstituted or which is substituted by at least one alkyl group having from 1 to 4 carbon atoms and/or alkoxy group having from 1 to 4 carbon atoms;
$R^3$ represents a hydrogen atom, an alkyl group having from 1 to 4 carbon atoms, an alkoxycarbonyl group having from 2 to 5 carbon atoms, an aralkyloxycarbonyl group in which the alkyl part has from 1 to 4 carbon atoms and the aryl part is as defined below, an aryloxycarbonyl group in which the aryl part is as defined

below, an aliphatic carboxylic acyl group having from 1 to 5 carbon atoms, an aliphatic carboxylic acyl group which has from 2 to 5 carbon atoms and which is substituted by at least one halogen atom, an arylcarbonyl group in which the aryl part is as defined below, an alkylsulphonyl group having from 1 to 4 carbon atoms, an arylsulphonyl group in which the aryl part is as defined below, or a group of formula -B-$NR^5R^6$, in which

B represents an alkylene or alkylidene group having from 2 to 4 carbon atoms; and

$R^5$ and $R^6$ are the same or different and each represents a hydrogen atom or an alkyl group having from 1 to 4 carbon atoms;

said aryl groups have from 6 to 10 ring atoms and are unsubstituted or are substituted by at least one alkyl group having from 1 to 4 carbon atoms, and/or alkoxy group having from 1 to 4 carbon atoms and/or halogen atom;

$R^4$ represents a hydrogen atom or an alkyl group having from 1 to 4 carbon atoms;

A represents an alkylene or alkylidene group having from 2 to 7 carbon atoms; and

Z represents a group of formula:

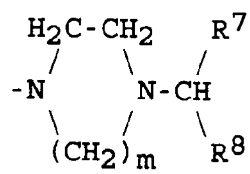

(II)

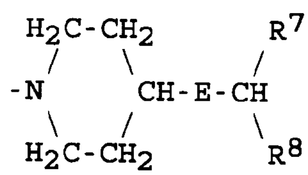

(III)

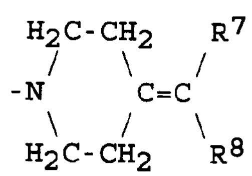

(IV)

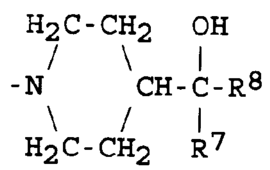

(V)

or a group of formulae (II), (III), (IV) or (V) in which one or more of the ring atoms is substituted by an alkyl group having from 1 to 4 carbon atoms;

in which:

$R^7$ and $R^8$ are the same or different and each represents an unsubstituted phenyl group, a substituted phenyl group which is substituted by at least one of substituents (a), defined below, or an aromatic heterocyclic group which has 5 or 6 ring atoms of which 1 or 2 are nitrogen and/or oxygen and/or sulphur hetero-atoms, said aromatic heterocyclic groups being unsubstituted or being substituted by at least one of substituents (a), defined below;

said substituents (a) are selected from alkyl groups having from 1 to 4 carbon atoms, alkoxy groups having from 1 to 4 carbon atoms, halogen atoms and haloalkyl groups having from 1 to 4 carbon atoms;

E represents a direct carbon-carbon single bond or an oxygen atom; and

m is 2 or 3];

or a pharmaceutically acceptable salt thereof, which comprises reacting a compound of formula (VI):

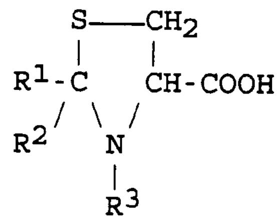

(VI)

(in which $R^1$, $R^2$ and $R^3$ are as defined above) or a reactive derivative thereof with a compound of formula (VII):

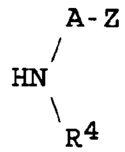

(VII)

(in which A, Z and $R^4$ are as defined above) or a compound of formula (VI) or (VII) or reactive derivative thereof in which a reactive group in one or more of the substituents Z, $R^1$, $R^2$, $R^3$ and $R^4$ is protected, and, if desired, removing protecting groups from the resulting product, and, if desired, salifying the compound of formula (I).

2. A process according to Claim 1, in which the reagents and reaction conditions are so chosen as to prepare a compound of formula (I) or a salt thereof, in which $R^1$ represents an unsubstituted pyridyl group or a substituted pyridyl group having at least one alkyl substituent having from 1 to 4 carbon atoms.

3. A process according to Claim 1 or Claim 2, in which the reagents and reaction conditions are so chosen as to prepare a compound of formula (I) or a salt thereof, in which $R^2$ represents a hydrogen atom.

4. A process according to any one of Claims 1 to 3, in which the reagents and reaction conditions are so chosen as to prepare a compound of formula (I) or a salt thereof, in which $R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms.

5. A process according to Claim 4, in which the reagents and reaction conditions are so chosen as to prepare a compound of formula (I) or a salt thereof, in which $R^3$ represents a hydrogen atom.

6. A process according to any one of Claims 1 to 5, in which the reagents and reaction conditions are so chosen as to prepare a compound of formula (I) or a salt thereof, in which $R^4$ represents a hydrogen atom.

7. A process according to any one of Claims 1 to 6, in which the reagents and reaction conditions are so chosen as to prepare a compound of formula (I) or a salt thereof, in which Z represents a 4-(diphenylmethyl)-1-piperazinyl, 4-[α-(chlorophenyl)benzyl]-1-piperazinyl, 4-[bis(fluorophenyl)methyl]-1-piperazinyl, 4-[α-(chlorophenyl)-o-, m- or p-fluorobenzyl]-1-piperazinyl, 4-[bis(chlorophenyl)methyl]-1-piperazinyl, 4-(diphenylmethyl)-1-piperidyl, 4-[bis(fluorophenyl)methyl]-1-piperidyl, 4-[α-(chlorophenyl)benzyl]-1-piperidyl, 4-(diphenylmethoxy)-1-piperidyl, 4-[α-(fluorophenyl)benzyloxy]-1-piperidyl, 4-[bis(fluorophenyl)methoxy]-1-piperidyl, 4-[α-(chlorophenyl)benzyloxy]-1-piperidyl, 4-(diphenylmethylene)-1-piperidyl, 4-[α-(fluorophenyl)benzylidene]-1-piperidyl, 4-[bis(fluorophenyl)methylene]-1-piperidyl, 4-[α-(chlorophenyl)benzylidene]-1-piperidyl, 4-(α-hydroxydiphenylmethyl)-1-piperidyl, 4-[α-(fluorophenyl)-α-hydroxybenzyl]-1-piperidyl, 4-[bis(fluorophenyl)-α-hydroxymethyl]-1-piperidyl or 4-[α-(chlorophenyl)-α-hydroxybenzyl]-1-piperidyl group.

8. A process according to Claim 1, in which the reagents and reaction conditions are so chosen as to prepare a compound of formula (I) or a salt thereof, in which:
$R^1$ represents a pyridyl group which is unsubstituted or which is substituted by at least one alkyl substituent having from 1 to 4 carbon atoms;
$R^2$ represents a hydrogen atom;
$R^3$ represents a hydrogen atom, an alkyl group having from 1 to 4 carbon atoms, an alkoxycarbonyl group

having from 2 to 5 carbon atoms or an aliphatic carboxylic acyl group having from 1 to 5 carbon atoms;
$R^4$ represents a hydrogen atom or an alkyl group having from 1 to 4 carbon atoms;
A represents an alkylene or alkylidene group having from 2 to 7 carbon atoms; and
Z represents a group of formula (II), (III), (IV) or (V), as defined in Claim 1, in which:

$R^7$ and $R^8$ are the same or different and each represents an unsubstituted phenyl group, a substituted phenyl group which is substituted by at least one of substituents (a′), defined below, or an aromatic heterocyclic group which has 5 or 6 ring atoms of which 1 is a nitrogen and/or oxygen and/or sulphur hetero-atom, said aromatic heterocyclic group being unsubstituted or being substituted by at least one alkyl substituent having from 1 to 4 carbon atoms;

said substituents (a′) are alkyl groups having from 1 to 4 carbon atoms, halogen atoms or trifluoromethyl groups;

E represents an oxygen atom; and

m is 2.

**9.** A process according to Claim 1, in which the reagents and reaction conditions are so chosen as to prepare a compound of formula (I) or a salt thereof, in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a group of formula (II), (III), (IV) or (V), as defined in Claim 1, in which:

$R^7$ and $R^8$ are the same or different and each represents an unsubstituted phenyl group, a substituted phenyl group which is substituted by at least one of substituents (a″), defined below, a pyridyl group or a thienyl group;

said substituents (a″) are methyl groups, fluorine atoms or chlorine atoms;

E represents an oxygen atom; and

m is 2.

**10.** A process according to Claim 1, in which the reagents and reaction conditions are so chosen as to prepare a compound of formula (I) or a salt thereof, in which:
$R^1$ represents a pyridyl group which is unsubstitutedor which is substituted by at least one alkyl substituent having from 1 to 4 carbon atoms;
$R^2$ represents a hydrogen atom;
$R^3$ represents a hydrogen atom, an alkyl group having from 1 to 4 carbon atoms, an alkoxycarbonyl group having from 2 to 5 carbon atoms or an aliphatic carboxylic acyl group having from 1 to 5 carbon atoms;
$R^4$ represents a hydrogen atom or an alkyl group having from 1 to 4 carbon atoms;
A represents an alkylene or alkylidene group having from 2 to 6 carbon atoms; and
Z represents a group of formula (II), (III), (IV) or (V), in which:

$R^7$ and $R^8$ are the same or different and each represents an unsubstituted phenyl group or a substituted phenyl group which is substituted by at least one of substituents (a‴), defined below;

said substituents (a‴) are alkyl groups having from 1 to 4 carbon atoms or halogen atoms;

E represents a direct carbon-carbon single bond or an oxygen atom; and

m is 2 or 3.

**11.** A process according to Claim 1, in which the reagents and reaction conditions are so chosen as to prepare a compound of formula (I) or a salt thereof, in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a group of formula (II), (III), (IV) or (V), in which:

$R^7$ and $R^8$ are the same or different and each represents an unsubstituted phenyl group or a substituted phenyl group which is substituted by at least one of substituents (a″), defined below;

said substituents (a″) are methyl groups, fluorine atoms or chlorine atoms;

E represents an oxygen atom; and

m is 2.

**12.** A process according to Claim 1, in which the reagents and reaction conditions are so chosen as to prepare a compound of formula (I) or a salt thereof, in which:
$R^1$ represents an unsubstituted pyridyl group;
$R^2$ represents a hydrogen atom;
$R^3$ represents a hydrogen atom;
$R^4$ represents a hydrogen atom or a methyl or ethyl group;
A represents an alkylene or alkylidene group having from 2 to 4 carbon atoms; and
Z represents a 4-[α-(chlorophenyl)benzyl]-1-piperazinyl, 4-[bis(fluorophenyl)methyl]-1-piperazinyl, 4-(diphenylmethylene)-1-piperidyl, 4-[bis(fluorophenyl)methoxy]-1-piperidyl or 4-(α-hydroxydiphenylmethyl)-1-piperidyl group.

**13.** A process according to Claim 1, in which the reagents and reaction conditions are so chosen as to prepare:
{3-[4-bis(4-fluorophenyl)methyl-1-piperazinyl] propylcarbamoyl}-2-(3-pyridyl)thiazolidine;
{3-[4-(α-2-pyridylbenzyl)-1-piperazinyl]propylcarbamoyl}-2-(3-pyridyl)thiazolidine;
[2-(4-diphenylmethyl-1-piperazinyl)ethylcarbamoyl]-2-(3-pyridyl)thiazolidine;
[3-{4-[α-(4-chlorophenyl)benzyl]-1-piperazinyl} propylcarbamoyl]-2-(3-pyridyl)thiazolidine;
[2-{4-[α-(4-chlorophenyl)benzyl]-1-piperazinyl}ethylcarbamoyl]-2-(3-pyridyl)thiazolidine;
{3-[4-bis(4-fluorophenyl)methoxy-1-piperidyl]propylcarbamoyl}-2-(3-pyridyl)thiazolidine;
{2-[4-(diphenylmethylene)-1-piperidyl]ethylcarbamoyl}-2-(3-pyridyl)thiazolidine;
{3-[4-(diphenylmethylene)-1-piperidyl]propylcarbamoyl}-2-(3-pyridyl)thiazolidine;
{4-[4-(diphenylmethylene)-1-piperidyl]butylcarbamoyl}-2-(3-pyridyl)thiazolidine;
[3-{4-[bis(4-fluorophenyl)methylene]-1-piperidyl}propylcarbamoyl]-2-(3-pyridyl)thiazolidine;
{3-[4-(α-2-thienyl)benzylidene-1-piperidyl]propylcarbamoyl}-2-(3-pyridyl)thiazolidine;
[N-{3-[4-diphenylmethylene-1-piperidyl]propyl}-N-ethylcarbamoyl]-2-(3-pyridyl)thiazolidine;
[N-{2-[4-diphenylmethylene-1-piperidyl]ethyl}-N-methylcarbamoyl]-2-(3-pyridyl)thiazolidine; or
{2-[4-(α-hydroxydiphenylmethyl)-1-piperidyl]ethylcarbamoyl}-2-(3-pyridyl)thiazolidine;
or a salt thereof.

**14.** A process for preparing a composition for the treatment or prophylaxis of histamine- or PAF- related disorders in a mammal, which comprises mixing an anti-histamine or anti-PAF agent with a pharmaceutically acceptable carrier or diluent, in which the anti-histamine or anti-PAF agent is at least one compound as defined in any one of Claims 1 to 13.

**15.** The use of a compound as defined in any one of Claims 1 to 13 for the manufacture of a medicament for the treatment or prophylaxis of histamine-related disorders.

European Patent Office

# EUROPEAN SEARCH REPORT

Application Number

EP 91 30 5804

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 350 145 (YAMANOUCHI PHARMACEUTICAL CO.)<br>* Claims *<br>--- | 1,14-16 | C 07 D 417/04<br>C 07 D 417/14<br>A 61 K 31/44<br>A 61 K 31/445 |
| A | EP-A-0 279 681 (YAMANOUCHI PHARMACEUTICAL CO.)<br>* Claims *<br>--- | 1,14-16 | |
| A | EP-A-0 258 033 (PFIZER LTD)<br>* Claims; page 2 *<br>----- | 1,14-16 | |
| | | | **TECHNICAL FIELDS SEARCHED (Int. Cl.5)** |
| | | | C 07 D 417/00 |

The present search report has been drawn up for all claims

| Place of search | Date of completion of the search | Examiner |
|---|---|---|
| THE HAGUE | 24-09-1991 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)